# EUROPÄISCHE PATENTSCHRIFT

(11) **EP 2 649 043 B1**
(45) Veröffentlichungstag und Bekanntmachung des Hinweises auf die Patenterteilung: **15.11.2017**
(21) Anmeldenummer: 11788107.8
(22) Anmeldetag: 18.11.2011
(51) Int. Cl.: C07C 209/26, C07C 253/10, C07C 209/48, C07C 45/74

(54) **VERFAHREN ZUR HERSTELLUNG VON 3-AMINOMETHYL-3,5,5-TRIMETHYLCYCLOHEXYLAMIN**
PROCESS FOR PREPARING 3-AMINOMETHYL-3,5,5-TRIMETHYLCYCLOHEXYLAMINE
PROCÉDÉ DE PRÉPARATION DE 3-AMINOMÉTHYL-3,5,5-TRIMÉTHYLCYCLOHEXYLAMINE

(30) Priorität: 08.12.2010 DE 102010062603
(43) Veröffentlichungstag der Anmeldung: 16.10.2013
(73) Patentinhaber: Evonik Degussa GmbH, 45128 Essen (DE)
(72) Erfinder: GALLE, Markus, 44357 Dortmund (DE); GRUND, Gerda, 48653 Coesfeld (DE); HENGSTERMANN, Axel, 48308 Senden (DE); HINTON, Michael D., Theodore AL 36582 (US); HIRSCH, Rolf, 45770 Marl (DE); JANSEN, Robert, 46244 Bottrop (DE); KNOOP, Cord, 45721 Haltern am See (DE); LETTMANN, Christian, 48653 Coesfeld (DE); LIPPE, Juergen, 45891 Gelsenkirchen (DE); MAIER, Martin, 44625 Herne (DE); NITZ, Jörg-Joachim, 46047 Oberhausen (DE); ORSCHEL, Matthias, 48149 Münster (DE); ORTELT, Martina, 48249 Dülmen (DE); RICHTER, Norbert, 45772 Marl (DE); RIX, Armin, 45770 Marl (DE); SCHWARZ, Markus, 45721 Haltern am See (DE); STREUKENS, Guido, 42111 Wuppertal (DE)
(86) Internationale Anmeldenummer: PCT/EP2011/070442
(87) Internationale Veröffentlichungsnummer: WO 2012/076317

(56) Entgegenhaltungen:
- EP-A1- 0 394 967
- DE-B- 1 240 854
- US-A- 5 055 620

## Beschreibung

Die Erfindung betrifft ein verbessertes Verfahren zur Herstellung von 3-Aminomethyl-3,5,5-trimethylcyclohexylamin, nachfolgend Isophorondiamin oder abgekürzt IPDA genannt, durch
I. Herstellung von Isophoron durch katalysierte Aldol-Kondensationen mit Aceton als Edukt
II. Umsetzung von Isophoron mit HCN unter Bildung von Isophoronnitril (IPN, 3-Cyano-3,5,5-Trimethylcyclohexanon);
III. katalytische Hydrierung und/oder katalytische reduktive Aminierung (auch als aminierende Hydrierung bezeichnet) von 3-Cyano-3,5,5-trimethylcyclohexanon, nachfolgend Isophoronnitril oder abgekürzt IPN genannt, zum Isophorondiamin.

### Stand der Technik zu I.

Isophoron wird u. a. als hochsiedendes Lösemittel in der Lack-, Druckfarben-, Klebstoff- und der Pflanzenschutzmittel-Industrie verwendet. Nach dem bekannten Stand der Technik kann das Isophoron weiter verarbeitet werden bspw. zu Isophoronnitril, Isophorondiamin, Isophorondiisocyanat oder Keto-Isophoron.

Bei Isophoron handelt es sich um das trimere Kondensationsprodukt des Acetons. Die Herstellung von Isophoron erfolgt über eine katalysierte Aldol-Kondensation von Aceton.

Sowohl die bestehende Patentliteratur als auch die wissenschaftlichen Veröffentlichungen zur Herstellung von Isophoron lassen sich im Wesentlichen in zwei Bereiche einteilen. Man unterscheidet zwischen Flüssigphasen- und Gasphasenprozessen. In CN 101633610A wird auch die Kondensationsreaktion zur Herstellung von Isophoron mit überkritischem Aceton beschrieben.

Während in den beschriebenen Gasphasenprozessen zum größten Teil mit heterogenen, festen Katalysatoren gearbeitet wird, kommen in Flüssigphasenprozessen sowohl homogene als auch heterogene Katalysatorsysteme zum Einsatz.

Die Reaktion in der Flüssigphase wird in der Patentliteratur nahezu ausschließlich unter alkalischen Bedingungen bei erhöhten Temperaturen und hohen Drücken beschrieben.

Auf dem Gebiet der Isophoronchemie sind mehrere Patente der Shell Development Company bekannt (US 2,351,352, US 2,344,226, US 2,399,976, US 2,419,051). Unter anderem wird in der US 2,399,976 ein Kondensationsverfahren zur Herstellung von Isophoron im Kreislaufreaktor mittels Alkalikatalyse beschrieben. Die verwendete Lauge wird in dem Verfahren nach Phasentrennung wieder in den Reaktor zurückgeführt, während das entstandene Reaktionswasser mit der organischen Phase aus dem Reaktorkreislauf entfernt wird.

Des Weiteren ist in US 2,419,051 ein Verfahren beschrieben, in dem durch Hydrolyse der höheren Kondensationsprodukte ein Teil der Überkondensate zurückgespalten werden kann. Die Hydrolyse wird in einem Druckreaktor bei Temperaturen zwischen 130 - 235 °C mit einer erhöhten Laugekonzentration durchgeführt.

Um in der Synthese eine Phasentrennung zu verhindern, und somit eine einphasige Reaktionsführung zu erreichen, sind in den Anmeldungen der Societe Industrielle Des Derivatives De L'Acetylene (DE 10 58 047, GB733650) Alkohole als Lösungsvermittler beschrieben. Dieses Verfahren führt zu einer geringeren Reaktionszeit. Des Weiteren ist dort beschrieben, dass die Rückführung von abgetrennten Nebenprodukten in die Reaktionszone des Reaktors die Selektivität der Isophoronbildung steigert.

In den Patentdokumenten der Hibernia Chemie (DE 10 95 818, DE 11 44 269, DE 12 05 525, DE 11 65 018) aus den 1960er Jahren wird neben dem Einsatz eines einphasigen Edukt / Katalysator-Gemisches mit niedrigen Lauge-Konzentrationen auch die Aufarbeitung mittels Hydrolysekolonne beschrieben. Isophoron wird hier in einem Druckreaktor durch Kondensation von Aceton in flüssiger Phase mittels Alkalimengen (NaOH oder KOH) von weniger als 1 % als Katalysator und unter Anwendung von Wassermengen von unterhalb 20 % bei Temperaturen von 150 - 250 °C hergestellt. Die sich bei der Reaktion ausbildenden zwei Phasen werden sowohl durch eine geeignete Reaktionsführung (Reaktorbau, Impulsgenerator), als auch durch den Einsatz eines Emulgators emulgiert, um einen guten Kontakt zwischen Katalysator und den Reaktanten zu gewährleisten (DE 10 95 818).

Daneben wird in DE 12 05 525 die Aufarbeitung von Nebenprodukten, der sog. Überkondensate beschrieben. Bei 120 - 300 °C findet mit einer wässrigen Alkali-Lösung in einer sogenannten Druckdestillationskolonne unter laufender Entfernung des gebildeten Acetons die Hydrolyse der Überkondensate statt.

Die Gewinnung von reinem Isophoron aus Isophoron-haltigen Kondensationsprodukten gelingt durch eine Abtrennung der Leichtsieder durch Destillation unter demselben Druck, bei dem die Kondensation durchgeführt wird und durch eine weitere Aufarbeitung der noch bestehenden Überkondensate durch Destillation bei vermindertem Druck (DE 11 44 269).

Laut der Anmeldung von BP Chemicals lässt sich durch Verwendung von Kalilauge (KOH) anstelle des sonst üblichen Katalysators Natronlauge (NaOH) die Isophoronausbeute bei gleichbleibender Selektivität um bis zu 7 % steigern (DE 25 20 681).

Weiterhin ist beschrieben, dass die Produktqualität des Isophorons erhöht werden kann, indem man farbbildende Substanzen in einem Seitenstrom aus der Reaktionskolonne ausschleust, und diesen Strom durch Destillation und saure Umsetzung aufreinigt (DE 26 45 281).

Weiterhin existieren Anmeldungen zur Isophoron-Herstellung von Daicel Chemical Industries (JP 8245485, JP 8245486) aus den 1990er Jahren. Diese beschreiben, dass durch Erniedrigung der Wasserkonzentration im Eduktstrom als auch durch Rückführung der wässrigen Laugephase nach der Phasentrennung in den Hydrolyseteil der Reaktivdestillation der Isophoronumsatz gesteigert werden kann.

Neben den bisher genannten Flüssigphasenprozessen mittels homogener Katalysatorsysteme gibt es auch eine Patent-Veröffentlichung mit heterogenen Katalysatorsystemen in der Flüssigphase.

So beschreibt Elf Atochem S. A. in dem Patent US 5,849,957 die Verwendung von Hydrotalciten (Mg₁₋ₓAlₓO₁₊ₓ) als heterogenes Katalysatorsystem für die Herstellung von Isophoron. In diskontinuierlichen Rührkesselversuchen konnten mit einem solchen Katalysator ein Acetonumsatz von 38 % und eine Selektivität zum Isophoron von 51 % erreicht werden.

Im Stand der Technik wird häufig die Herstellung von Isophoron mittels heterogener Katalysatoren auch in der Gasphase beschrieben.

In den Dokumenten der Union Carbide (US 4,086,188, US 4,165,339, EP 095 783) wird die Herstellung von Isophoron mittels Lithium-, bzw. Zink-dotierter Hydrotalcit-artiger Fällungskatalysatoren beschrieben. Mit diesen Katalysatoren kann bei einem Acetonumsatz von 24 % eine Selektivität von 47 % bezüglich Isophoron erreicht werden (US 4,086,188) und der Katalysator durch Abbrennen der Verkokungsrückstände vollständig regeneriert werden (US 4,165,339). Durch Optimierung der Präparationsbedingungen kann die Standzeit eines solchen Katalysators auf bis zu ca. 1000 Stunden erhöht werden (EP 095 783).

In den Patenten der Aristech Chemical Corporation (WO9012645, WO9507255) sind verschiedene oxidische Magnesium/Aluminium-Katalysatoren beschrieben, die durch Aufschlämmung von Pseudoboehmit und Magnesiumoxid hergestellt werden (WO 9012645). Bei einem Aceton Umsatz von 30 % liegt die Selektivität bezüglich Isophoron bei 76 %. Neben den Katalysatoren beschreibt die Aristech Chemical Company auch ein Verfahren zur Herstellung von Isophoron in der Gasphase in einem Festbettreaktor (WO 95072559). Der Acetonumsatz wird hierbei auf 10 - 35 % begrenzt, um die Bildung von Verkokungsrückständen zu minimieren.

Weiterhin gibt es eine Reihe von Anmeldungen (JP 9059204, JP 9151152, JP 9151153, JP 9157207, JP 9157208, JP 9169687, JP 9169688) von Mitsui Toatsu Chemicals, die verschiedene Zeolith- und Magnesium/Alkalimetall-Katalysatoren für die Herstellung von Isophoron beanspruchen.

In wissenschaftliche Veröffentlichungen ist neben den bereits in den Patenten genannten Katalysatorsystemen ebenfalls die Verwendung von Kohlenstoff-Nanoröhrchen als Katalysator für die Isophoron-Synthese beschrieben. M. G. Stevens (Chem. Commun. 3, 1999) erreicht mit Cäsium-dotierten Kohlenstoff-Nanoröhrchen einen AcetonUmsatz von 11.9 % bei einer Isophoron-Selektivität von 61 %.

Bei der Synthese von Isophoron entsteht eine ganze Reihe von unerwünschten Nebenprodukten. Diese sind bspw. Diacetonalkohol, Mesityloxid, Phoron, Mesitylen sowie eine Reihe von höheren Kondensationsprodukten (Überkondensate) des Acetons (z. B. Xylitone und Isoxylitone). Aus diesem Grund ist die Erzielung von hohen Ausbeuten und Selektivitäten an Isophoron schwierig zu erreichen.

### Stand der Technik zu II.

Die Basen katalysierte Umsetzung von Blausäure (HCN) mit alpha, beta-ungesättigten cyclischen (oder acyclischen) Ketonen ist eine bekannte Reaktion (Hydrocyanation of Conjugated Carbonyl Compounds, CHAPTER 3, Wataru Nagata and Mitsuru Yoshioka).

Weitere Verfahren sind weiter unten genannt.

### Stand der Technik zu III.

Die Herstellung von IPDA durch aminierende Hydrierung von IPN ist bekannt und bereits mehrfach beschrieben worden.

Im einfachsten Fall (US 3,352,913) wird IPN in Gegenwart von Wasserstoff und eines Überschusses an Ammoniak an einem Cobaltkatalysator zur Reaktion gebracht. Zunächst bildet sich aus IPN und Ammoniak durch Wasserabspaltung das Isophoronnitrilimin, IPNI, das nachfolgend zum IPDA hydriert wird.

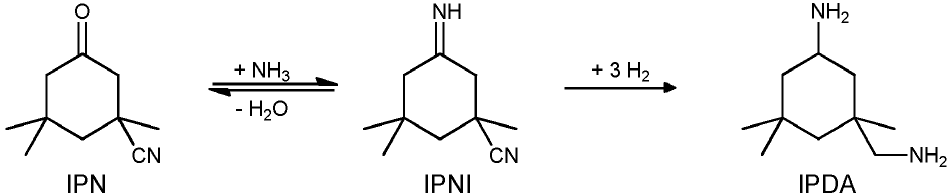

Die Ausbeute an IPDA wird bei dieser Art der Reaktionsführung maßgeblich durch den Überschuss an Ammoniak bestimmt. Die maximal erzielten IPDA-Ausbeuten liegen bei ca. 80 %. Hauptnebenprodukt ist der sogenannte Aminoalkohol, IPAA, der aus der direkten Hydrierung des IPN resultiert.

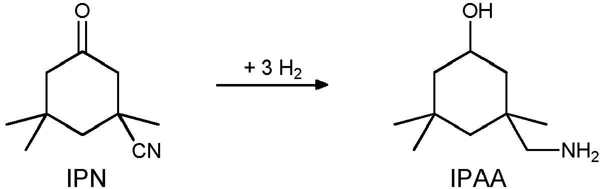

Eine wesentliche Steigerung der IPDA-Ausbeute wird erreicht, wenn die Bildung des IPNI durch Einsatz geeigneter Iminierungskatalysatoren beschleunigt wird. Als Iminierungskatalysatoren sind z.B. saure Ionenaustauscherharze (EP 042 119) geeignet. Daneben können auch acide Metalloxide (EP 449 089), sulfonsäuregruppenhaltige Organopolysiloxane (EP 816 323), Heteropolysäuren (DE 44 26 472) und Aktivkohle (EP 061 137) als Iminierungskatalysatoren eingesetzt werden. Neben der Reduzierung des unerwünschten Aminoalkohols werden auch andere Nebenprodukte deutlich zurück gedrängt, z. B. bicyclische Verbindungen und solche Nebenprodukte, die aus der Abspaltung von HCN resultieren.

Auf die Problematik der Abspaltung von HCN aus gamma-Ketonitrilen, wie dem IPN, wird in der Literatur besonders hingewiesen (US 3,352,913). Zum Einen wird bemerkt, dass durch die HCN-Abspaltung die Ausbeute an IPDA reduziert wird (EP 042 119, DE 44 26 472).

Zum Anderen wird darauf hingewiesen, dass HCN als Katalysatorgift wirkt und zu einer Desaktivierung des Hydrierkatalysators führt (EP 394 967 A1, Seite 2 Zeile 34 ff, Seite 3 Zeile 44 ff). Es wird daher empfohlen, den Iminierungsschritt so durchzuführen, dass möglichst kein HCN abgespalten wird. Bevorzugt soll der Prozess so gefahren werden, dass weniger als 0,001 mol HCN pro Mol eingesetztem Nitril abgespalten werden (EP 394 967 Seite 5 Zeile 49 ff). Bezogen auf die aminierende Hydrierung von IPN sind das 163 ppmw (0,0163 Gewichtsprozente).

Neben der Reduktion der Cyanidkonzentration sind weitere Methoden beschrieben, um bei der aminierenden Hydrierung von IPN IPDA die Ausbeute an IPDA zu erhöhen.

Wie bereits weiter oben erwähnt wirkt sich ein Überschuss an Ammoniak oder die Verwendung von Ammoniak als Lösungsmittel positiv auf die Ausbeute aus (z. B. EP 449 089, EP 659 734, DE 12 29 078).

Auch die Modifizierung mit Alkalihydroxiden (EP 729 937) führt zu einer Erhöhung der IPDA-Ausbeute. Dass durch den Zusatz von Alkalihydroxiden, vor allem Lithiumhydroxid, bei Nitrilhydrierungen die Ausbeute an primärem Amin gesteigert werden kann, ist aus mehreren Publikationen bekannt (US 4,375,003, EP 913 388). Die Katalysatoren können entweder vor der Reaktion mit Alkalihydroxiden behandelt werden, oder aber das Alkalihydroxid wird während der Reaktion der Reaktionsmischung zugesetzt. So lange keine größeren Mengen Lösungsmittel wie Ammoniak, THF oder Methanol verwendet werden, ist die Langzeitstabilität der LiOH-modifizierten Katalysatoren recht gut. In eigenen Versuchen mussten wir jedoch feststellen, dass bei Verwendung oben genannter Lösungsmittel das LiOH kontinuierlich vom Katalysator herunter gewaschen wird und somit der Anteil an sekundären Aminen wieder steigt. Bei einer kontinuierlichen Prozessführung, bei der das Lösungsmittel durch Destillation von der Mischung abgetrennt und in den Prozess zurückgeführt wird, kommt es zudem zu einer Ablagerung der Alkalihydroxide in den Destillationskolonnen. Die Kolonnen müssen in regelmäßigen Abständen abgestellt und gereinigt werden, sodass das die Alkalimodifizierung indirekt zu Produktionsausfällen führt.

Gemäß EP 913 387 können zur Selektivitätssteigerung bei der Herstellung von IPDA auch quaternäre Ammoniumbasen eingesetzt werden. Entsprechend modifizierte Katalysatoren weisen speziell bei Verwendung eines Lösungsmittels eine deutlich höhere Standzeit auf als alkalimodifizierte Katalysatoren.

Die Aufgabe dieser Erfindung war es, ausgehend von Aceton, ein verbessertes Verfahren zur Herstellung von 3-Aminomethyl-3,5,5-trimethylcyclohexylamin zu finden.

Die technische Aufgabe dieser Erfindung war es daher, ein Verfahren zu finden, das es ermöglicht, die Wirtschaftlichkeit bei der Isophoron Herstellung zu erhöhen. Dabei sollten auch ökologische Aspekte berücksichtigt sein.

Eine weitere Aufgabe der vorliegenden Erfindung war es, ein Verfahren zu Selektivitätssteigerung bei der katalytischen Hydrierung und/oder der katalytischen reduktiven Aminierung von IPN zu IPDA zu finden, das die genannten Nachteile der vorangehend beschriebenen Verfahren beseitigt.

Es wurde gefunden, dass das entstehende Reaktionsgemisch aus Schritt I. durch das erfinderische Verfahren besonders wirtschaftlich und ökologisch zu Isophoron aufgearbeitet werden kann.

Überraschend wurde nun ebenfalls gefunden, dass die Aufgabe durch eine Erhöhung der Cyanidionenkonzentration in der Reaktionsmischung im Schritt III., beispielsweise hervorgerufen durch die gezielte Abspaltung von HCN vom IPN, gelöst werden kann. Dies ist insofern überraschend, da Cyanidionen als Katalysatorgifte beschrieben sind und somit gemäß Stand der Technik zur Ausbeuteoptimierung und Selektivitätsoptimierung eine möglichst geringe Konzentration von Cyanidionen anzustreben ist.

Die Erhöhung der Cyanidionenkonzentration in einem bestimmten Bereich sorgt überraschender Weise bei der Hydrierung von IPNI zum IPDA für eine Selektivitätssteigerung bei gleichbleibendem Umsatz.

Gegenstand der Erfindung ist ein Verfahren zur Herstellung von 3-Aminomethyl-3,5,5-trimethylcyclohexylamin, durch
I. Herstellung von Isophoron durch katalysierte Aldol-Kondensationen mit Aceton als Edukt, Aufarbeitung des Reaktionsproduktes,
   Hydrolyse des Wertstroms und Trennung in eine organische und eine wässrige Fraktion, Gewinnung von Isophoron aus der organischen Fraktion,
   destillative Aufarbeitung der wässrigen Fraktion und Weiterleitung der Brüden vom Kopf der Apparatur der destillativen Aufarbeitung in die Hydrolyse Apparatur; wobei das Wasser aus dem Sumpf der destillativen Aufarbeitung der wässrigen Fraktion einer Entspannungsverdampfung unterworfen wird und das entstehende gereinigte Wasser in den Prozess zur Herstellung von Isophoron zurückgeführt wird;
II. Basenkatalysierte Umsetzung von Isophoron mit HCN unter Bildung von Isophoronnitril;
III. Herstellung von Isophorondiamin, mittels katalytischer Hydrierung und/oder katalytischer reduktiver Aminierung von Isophoronnitril (IPN), in Gegenwart von Ammoniak, Wasserstoff und mindestens eines Katalysators sowie gegebenenfalls eines Lösungsmittels oder Lösungsmittelgemisches, wobei die Cyanidionenkonzentration im Reaktionsgemisch, das der Hydrierung zugeführt wird von 200 ppmw bis 5000 ppmw beträgt, bezogen auf das eingesetzte Isophoronnitril, wobei die Herstellung von IPDA durch einen zweistufigen Prozess erfolgt,und wobei in der ersten Stufe zumindest ein Teil des eingesetzten Isophoronnitril bei An- oder Abwesenheit eines Iminierungskatalysators und/oder von Lösungsmitteln durch Reaktion mit Ammoniak in Isophoronnitrilimin umgewandelt wird, wobei der Umsatz von IPN zu IPNI nach der Iminierung größer 80 %, bevorzugt größer 90 %, besonders bevorzugt größer 95 % beträgt,undwobei in der zweiten Stufe das Reaktionsprodukt der ersten Stufe, so wie es anfällt oder nach einer Weiterbehandlung und/oder Zugabe weiteren Ammoniaks, unter Anwesenheit von mindestens Ammoniak und Wasserstoff und in An- oder Abwesenheit eines organischen Lösungsmittel bei einer Temperatur von 20 bis 150°C bevorzugt 40 bis 130 °C, und einem Druck von 0,3 bis 50 MPa, bevorzugt 5 bis 30 MPa, an Hydrierkatalysatoren aminierend hydriert wird.

### I: Herstellung von Isophoron

Im ersten Schritt des erfindungsgemäßen Verfahrens wird erfindungsgemäß Isophoron durch katalysierte Aldol-Kondensationen mit Aceton als Edukt hergestellt.
Die folgenden Angaben beziehen sich demnach auf diesen Schritt I.

Erfindungsgemäß wird in Schritt I. das Verfahren zur Herstellung von Isophoron durch katalysierte Aldol-Kondensationen mit Aceton als Edukt,
Aufarbeitung des Reaktionsproduktes,
Hydrolyse des Wertstroms und Trennung in eine organische und eine wässrige Fraktion, Gewinnung von Isophoron aus der organischen Fraktion, destillative Aufarbeitung der wässrigen Fraktion und Weiterleitung der Brüden vom Kopf der Apparatur der destillativen Aufarbeitung in die Hydrolyse Apparatur,
durchgeführt.

Weiterhin ist zusätzlivh Gegenstand der Erfindung gemäß Schritt I. ein Verfahren zur Herstellung von Isophoron wobei
das Wasser aus dem Sumpf der destillative Aufarbeitung der wässrigen Fraktion einer Entspannungsverdampfung unterworfen wird und das entstehende gereinigte Wasser in den Prozess zur Herstellung von Isophoron zurückgeführt wird.

Das erfinderische Verfahren im Schritt I. kann kontinuierlich, diskontinuierlich oder semikontinuierlich durchgeführt werden. Es wird jedoch in bevorzugter Weise kontinuierlich durchgeführt.

Die Herstellung von Isophoron erfolgt über katalysierte Aldol-Kondensationen mit Aceton als Edukt. Dabei reagieren im ersten Schritt zwei Aceton-Moleküle über das Zwischenprodukt Diacetonalkohol unter Wasserabspaltung zu Mesityloxid. In einer Folgereaktion reagiert das Mesityloxid mit einem weiteren Aceton wiederum unter Wasserabspaltung zum Isophoron.

Bei Isophoron handelt es sich also um das Reaktionsprodukt einer Kondensation von drei Molekülen Aceton unter der Abspaltung von zwei Molekülen Wasser.

Als Folge der chemischen Ähnlichkeit des eingesetzten Edukts (Aceton) und der gebildeten (Zwischen-) Produkte verläuft die Isophoron-Synthese nicht allzu selektiv. Aufgrund der Vielzahl von konkurrierenden Aldol-Kondensationsreaktionen erhält man unter Reaktionsbedingungen neben dem gewünschten Zielmolekül Isophoron sowohl eine ganze Reihe von unerwünschten (höheren) Kondensationsprodukten (z. B. Xylitone und Isoxylitone) als auch weitere Nebenkomponenten (z. B. Mesitylen).

Die Isophoron-Synthese ist also durch ein komplexes Reaktionsnetzwerk gekennzeichnet; die Selektivität ist in hohem Maße vom Umsatz abhängig. Um die Bildung von unerwünschten (höheren) Kondensationsprodukten zu minimieren, muss der Acetonumsatz begrenzt werden. Insbesondere in der Gasphasenreaktion kann der verwendete Katalysator durch sich bildende Verkokungsrückstände desaktiviert werden.

Es wurde gefunden, dass das entstehende Reaktionsgemisch durch das erfinderische Verfahren im Schritt I. besonders wirtschaftlich und ökologisch zu Isophoron aufgearbeitet werden kann.

Die Kondensationsreaktion von Aceton zu Isophoron (Reaktion) wird bevorzugt in einer katalysierten Flüssigphasenreaktion durchgeführt. Alternativ lässt sich Isophoron auch mittels einer Gasphasenreaktion oder auch durch Reaktion in überkritischem Aceton herstellen.

Für die Durchführung der Reaktion im Schritt I. gemäß dem erfindungsgemäßen Verfahren in der Flüssigphase wird das Aceton innerhalb des verwendeten Reaktors durch katalytische Reaktion bei Temperaturen im Bereich von 100 bis 250 °C, bevorzugt 150 - 250 °C, besonders bevorzugt 180 - 250 °C und einem Druckbereich von 5 bis 50 bar, bevorzugt 10 - 50 bar, besonders bevorzugt von 20 - 50 bar zu Isophoron umgesetzt, wobei die angegebenen Werte beliebig miteinander kombiniert werden können.

Für die Durchführung der Reaktion im Schritt I. gemäß dem erfindungsgemäßen Verfahren in der Gasphase wird das Aceton innerhalb des verwendeten Reaktors durch katalytische Reaktion bei Temperaturen im Bereich von 100 bis 400 °C, bevorzugt 200 - 400 °C zu Isophoron umgesetzt.

Für die Durchführung der Reaktion im Schritt I. gemäß dem erfindungsgemäßen Verfahren im überkritischen Bereich wird das Aceton innerhalb des verwendeten Reaktors durch katalytische Reaktion bei Temperaturen im Bereich von 250 bis 350 °C und einem Druckbereich von 50 bis 200 bar zu Isophoron umgesetzt.

Die katalytische Reaktion kann mit den im Stand der Technik genannten Katalysatoren durchgeführt werden, dabei kann es sich entweder um einen homogenen oder einen heterogenen Katalysator handeln. In der Flüssigphase wird bevorzugt ein homogener Katalysator eingesetzt, in der Gasphase bevorzugt ein heterogener Katalysator verwendet. Für die Reaktion im überkritischen Bereich können sowohl homogene als auch heterogene Katalysatoren verwendet werden.

Bei der bevorzugten Reaktion in der Flüssigphase lässt sich Isophoron mittels homogenem Katalysator mit Alkalimengen (NaOH oder KOH) von < 1 Gew.-%, bevorzugt von < 0,5 Gew.-%, besonders bevorzugt < 0,2 Gew.-%, herstellen. Besonders bevorzugt wird als Katalysator NaOH in Mengen von 0,015 bis 0,05 Gew.-% eingesetzt. Die eingesetzte Wasserkonzentration ergibt sich u. a. aus den Rückführströmen der Aufarbeitungsprozesse, sie sollte bezogen auf die gesamte Flüssigkeitsmenge unterhalb < 40 %, bevorzugt < 30 % liegen.

Die Reaktion kann in beliebigen Reaktoren nach dem Stand der Technik, wie z.B. Rohrreaktoren, Rührkesseln, Rührkesselkaskaden, Festbettreaktoren, Druckdestillationsreaktoren bzw. Reaktivdestillationen, mikrostrukturierten Reaktoren, Schlaufenreaktoren usw. oder in Kombinationen aus beliebigen Reaktoren durchgeführt werden. Dabei ist die Wahl der Reaktoren nicht auf die genannte Auswahl beschränkt.

Der Begriff Druckdestillationsreaktor ist hier gleichzusetzen mit Apparaten, in denen eine Reaktivdestillation durchgeführt wird. Die Reaktivdestillation ist in der Fachliteratur hinlänglich beschrieben, z.B. in Ullmann's Encylcopedia of Industrial Chemistry (M. Sakuth, D. Reusch, R. Janowsky: Reactive Distillation © 2008 Wiley-VCH Verlag GmbH & Co KGaA, Weinheim, DOI: 10.1002/14356007.c22_c01.pub2). Hier und in der zitierten Literatur sind alle gängigen Prozesse und Apparate der Reaktivdestillation beschrieben. Wird im folgenden Text der Patentschrift der Begriff der Reaktivdestillationskolonne verwendet, so sind sämtliche Ausführungsformen der Reaktivdestillation, wie in der Literatur beschrieben, gemeint.

In bevorzugter Ausführung erfolgt die Reaktionsdurchführung in Reaktivdestillationskolonnen, Rohrreaktoren oder Festbettreaktoren. Besonders bevorzugt sind Rohrreaktoren.

Nach Durchführung der Reaktion wird das Reaktionsgemisch aufgearbeitet und in die einzelnen Komponenten getrennt. Diese sind neben Isophoron sogenannte Leichtsieder wie z. B. Aceton, Diacetonalkohol und Mesityloxid, sowie eine Reihe von höheren Kondensationsprodukten (Überkondensate) des Acetons (z. B. Xylitone und Isoxylitone), Wasser und gegebenenfalls Katalysator. Dabei wird die Trennung ganz oder teilweise durchgeführt.

Die Abtrennung der einzelnen Fraktionen kann mit allen Trennmethoden wie z. B. Destillation, Entspannungsverdampfung, Kristallisation, Extraktion, Sorption, Permeation, Phasentrennung oder Kombinationen aus den genannten, kontinuierlich oder absatzweise, ein- oder mehrstufig durchgeführt werden. Bevorzugt wird die Trennung durch Destillation in einem oder mehreren Apparaten erreicht.
Dabei kann die Destillation räumlich getrennt von der Isophoronsynthese (Reaktion) durchgeführt werden oder in einem Apparat stattfinden. Bevorzugt erfolgt die Abtrennung der einzelnen Fraktionen durch eine Reaktivdestillation, bevorzugt in einer Reaktivdestillationskolonne.

Besonders bevorzugt wird die Abtrennung räumlich getrennt von der Isophoronsynthese (Reaktion) in einer Reaktivdestillationskolonne mit einer Seitenstromentnahme durchgeführt.

Bevorzugt erfolgt die Abtrennung in drei Fraktionen:
a) Einer Fraktion aus nicht umgesetzten Aceton, Wasser und Leichtsiedern, wie z.B. Diacetonalkohol und Mesityloxid, die kondensiert und anschließend zur Reaktion in den Reaktor zurückgeführt wird.
b) Einer Fraktion, in der insbesondere farbgebende Substanzen angereichert werden. Diese Fraktion wird weiter aufgereinigt und die enthaltenden Wertstoffe werden im Prozess zurückgeführt.
c) Einer Fraktion aus insbesondere Isophoron, höherkondensierten Produkten und Wasser und gegebenenfalls Katalysator, genannt Wertstrom. Diese Fraktion wird anschließend einer Hydrolyse unterworfen.

Die Fraktion a) wird in der bevorzugten Ausführungsform als Brüdenstrom entnommen, enthaltend im Wesentlichen Aceton, Wasser und Leichtsieder, im Wesentlichen Diacetonalkohol und Mesityloxid, kondensiert und dem Reaktor mit den Einsatzstoffen Aceton, Wasser und gegebenenfalls Katalysator wieder zugesetzt.

Die Fraktion b) wird in der bevorzugten Ausführungsform als Seitenstrom der Destillationskolonne, bevorzugt einer Reaktivdestillationskolonne, entnommen, gegebenenfalls neutralisiert und weiter aufgearbeitet. Dabei können bei der Aufarbeitung alle gängigen Trennmethoden wie z.B. Destillation, Entspannungsverdampfung, Kristallisation, Extraktion, Sorption, Permeation, Phasentrennung oder Kombinationen aus den genannten zum Einsatz kommen. Die Aufreinigung kann kontinuierlich oder absatzweise, ein- oder mehrstufig durchgeführt werden. Bevorzugt wird die Aufreinigung durch Destillation erreicht. Besonders bevorzugt wird die Aufreinigung durch eine Kombination aus Neutralisation oder Extraktion und anschließender Destillation, bevorzugt in einer Reaktivdestillationskolonne, erreicht. Bevorzugt wird die aufgearbeitete Phase mit den Wertprodukten aus Isophoron, Hochsiedern und gegebenenfalls Katalysator in die Hydrolyse geführt. Eine weitere erhaltene Phase aus Wertprodukten, im Wesentlichen enthaltend Aceton, Diacetonalkohol und Mesityloxid, wird bevorzugt in die Reaktion zurückgeführt. Gegebenenfalls anfallende Rückstände werden der thermischen Verwertung zugeführt.

Die Fraktion c) wird einer Hydrolyse unterworfen. Das Ziel der Hydrolyse ist es, Nebenprodukte teilweise oder vollständig in Isophoron, Aceton und andere Wertprodukte zu überführen. Die Hydrolyse kann in allen gängigen Reaktoren, welche bereits oben beschrieben wurden, oder Destillationskolonnen oder Kombinationen aus beiden, durchgeführt werden. Bevorzugt wird die Hydrolyse durch eine Reaktivdestillation durchgeführt, in dem die entstehenden Leichtsieder, im Wesentlichen enthaltend Aceton, Diacetonalkohol und Mesityloxid, direkt aus der Hydrolysezone entfernt und in die Reaktion zurückgeführt werden, und somit nicht mehr für Nebenreaktionen in der Hydrolyse zur Verfügung stehen.

Ganz besonders bevorzugt wird die Hydrolyse der Fraktion c) in einem Apparat, durch eine Reaktivdestillation durchgeführt, bevorzugt in einer Reaktivdestillationskolonne, wobei gleichzeitig die Trennung des Reaktionsgemisches in die Fraktionen a) bis c) erfolgt, so dass die entstehenden Produkte gleichzeitig entsprechend aufgetrennt werden und die Fraktion c) hydrolysiert wird.

Gegebenenfalls können die Hydrolyse und die destillative Abtrennung auch in einem Apparat mit der Isophoronsynthese (Reaktion) stattfinden.

Die Hydrolyse kann in allen Mischungsverhältnissen der organischen Komponenten mit Wasser mit oder ohne Katalysator durchgeführt werden. Die Wasserkonzentration in der Hydrolyse beträgt dabei 0,1 - 99,9 Gew.-%, bevorzugt 30 - 90 Gew.-%. Im Falle der homogenen Katalyse wird bei der Hydrolyse bevorzugt derjenige Katalysator eingesetzt, der auch im Reaktionsteil verwendet wird. Bevorzugt werden Katalysatorkonzentrationen von 0,001 - 10 Gew.-%, besonders bevorzugt von 0,05 - 1 Gew.-%. Der Druck im Hydrolysereaktor beträgt 1-200 bar, bevorzugt 20 - 60 bar, besonders bevorzugt wird die Hydrolyse mindestens bei dem Druck durchgeführt, der auch im Isophoronsyntheseschritt (Reaktion) herrscht. Die Temperatur der Hydrolyse beträgt 100 - 300 °C, bevorzugt 210 - 260 °C. Besonders bevorzugt wird sich bei Verwendung einer Reaktivdestillationskolonne eine Temperatur bzw. ein Temperaturverlauf einstellen, der den Siedetemperaturen im Sumpf und auf den einzelnen Trenn- oder Reaktionsstufen entspricht.

Die Hydrolyse kann in einem oder mehreren Apparaten einstufig oder mehrstufig durchgeführt werden.

Die so aufgearbeitete Fraktion c) wird anschließend aus dem Hydrolysereaktor bzw. der Reaktivdestillationskolonne abgetrennt, abgekühlt und einer Phasentrennung unterworfen.

Die Phasentrennung erfolgt in eine im Wesentlichen organische Fraktion d) und eine im Wesentlichen wässrige Fraktion e), die, bei homogener Katalyse, auch den Katalysator enthält. Dabei können übliche Phasentrennbehälter mit und ohne Einbauten zum Einsatz kommen. Die Phasentrennung erfolgt bei einer Temperatur zwischen 0 - 200 °C, bevorzugt bei 0 - 100 °C, besonders bevorzugt bei 20 - 70 °C und einem Druck von 1 - 150 bar, bevorzugt 20 - 60 bar, besonders bevorzugt bei dem Druck, der auch in der Hydrolyse herrscht.

Die im Wesentlichen organische Fraktion d) mit dem Zielprodukt Isophoron wird gegebenenfalls neutralisiert und mit üblichen Methoden aufgereinigt, so dass ein Isophoron mit der gewünschten Reinheit und Farbstabilität erhalten wird. Dabei können alle gängigen Trennmethoden wie z.B. Destillation, Entspannungsverdampfung, Kristallisation, Extraktion, Sorption, Permeation, Phasentrennung oder Kombinationen aus den genannten zum Einsatz kommen. Die Aufreinigung kann kontinuierlich oder absatzweise, ein- oder mehrstufig, unter Druck oder im Vakuum durchgeführt werden. Bevorzugt wird die Aufreinigung durch Destillation erreicht. Besonders bevorzugt wird die Aufreinigung durch eine Kombination aus Neutralisation oder Extraktion und folgender Destillation erreicht.

An dieser Stelle wird die destillative Aufarbeitung der wässrigen Fraktion e) (Abwasseraufreinigung) und Weiterleitung der Brüden vom Kopf der Apparatur der destillativen Aufbereitung in die Hydrolyseapparatur genauer beschrieben.

Die im Wesentlichen wässrige Fraktion e) wird einer Abwasseraufreinigung zugeführt. Hier erfolgt die Trennung des Reaktionswassers als Hauptbestandteil und gegebenenfalls des Katalysators von gegebenenfalls noch gelösten organischen Komponenten, wie z. B. Isophoron, Aceton und höher kondensierten Produkten. Die Abwasseraufreinigung wird bevorzugt in einer oder mehreren Destillationskolonnen durchgeführt. Erfindungswesentlich ist dabei, dass die Brüden der Abwasserkolonne direkt in den Apparat geleitet werden, in dem die Hydrolyse stattfindet. Dadurch werden mehrere Probleme des derzeitigen Standes der Technik gleichzeitig gelöst:
1) Da die Brüden im Wesentlichen aus Wasser bestehen, stellt sich im Hydrolyseteil eine notwendige, ausreichend hohe Wasserkonzentration ein, so dass kein zusätzliches Frischwasser in die Hydrolyse eingebracht werden muss.
2) Die in der Fraktion e) gelösten, organischen Anteile werden teilweise oder komplett über die Brüden der Abwasserkolonne in den Prozess zurückgeführt. Das minimiert die organische Belastung im Abwasser und, da es sich im Wesentlichen um Isophoron handelt, erhöht die Gesamtausbeute im Prozess. Diese neuartige Verschaltung der Abwasserkolonne trägt somit einen wesentlichen Beitrag zur ökologischen und ökonomischen Prozessführung bei.
3) Außerdem wird die notwendige Wärme für die Hydrolyse bzw. die destillative Trennung des Reaktionsgemisches durch die Brüden zur Verfügung gestellt, es wird keine separate Heizung benötigt.

Der Druck in der Abwasserkolonne beträgt 1 - 200 bar, bevorzugt 20 - 60 bar. Besonders bevorzugt wird bei dem Systemdruck gearbeitet, der sich im Gesamtsystem Hydrolyse/Abwasserkolonne einstellt, wenn die Brüden der Abwasserkolonne direkt in den Hydrolyseteil der Reaktivdestillation geleitet werden. Die Temperatur in der Abwasserkolonne entspricht der Siedetemperatur der Fraktion e) unter den Druckbedingungen. Die bevorzugte Temperatur der Brüden beträgt 200 - 300 °C.

Im Folgenden wird näher beschrieben, wie das Wasser aus dem Sumpf der destillativen Aufarbeitung der wässrigen Fraktion einer Entspannungsverdampfung unterworfen wird und das entstehende gereinigte Wasser in den Prozess zur Herstellung von Isophoron zurückgeführt wird.

Das im Sumpf der Abwasserkolonne anfallende Abwasser (Strom f) kann abgekühlt und verworfen werden. Bevorzugt wird das Abwasser f) aber einer Entspannungsverdampfung zugeführt und somit weiter aufgetrennt. Die Brüden g) der Entspannungverdampfungsstufe, die im Wesentlichen aus reinem Wasser bestehen, können kondensiert und als Wasser in den Prozess, bevorzugt in die Reaktion, z. B. zur Verdünnung des eingesetzten Katalysators (im Falle der homogenen Katalyse) zurückgeführt werden. Dadurch wird die Abwassermenge nochmals reduziert. Die Entspannungsverdampfung kann ein- oder mehrstufig kontinuierlich oder diskontinuierlich durchgeführt werden. Der Druck in der Entspannungsverdampfung liegt in jedem Fall unterhalb des Druckes in der Abwasserkolonne. Bevorzugt ist bei dem erfindungsgemäßen Verfahren die Anwendung einer Entspannungsverdampfung.

Sämtliche Destillations- und Reaktionsschritte im Prozess können in Reaktoren oder Apparaten mit oder ohne Einbauten durchgeführt werden, wie z. B. Dephlegmatoren, ungeordnete Einbauten oder Schüttungen, geordnete Einbauten oder Packungen, Böden mit oder ohne Zwangsführung.

Alle für die Reaktion eingesetzten produktberührten metallischen Werkstoffe und die aus den metallischen Werkstoffen hergestellten Apparate sowie deren Einbauten müssen gegen Laugen beständig sein. Dabei können in Abhängigkeit von der Gefährdung unterschiedliche Beständigkeitsanforderungen bestehen. Für die Beständigkeiten sind nicht nur die chemischen und/oder mechanischen Eigenschaften von Bedeutung, sondern auch die zur Anwendung kommenden Fertigungsmethoden und die Beurteilungsmaßstäbe während der Prüfung.

Für die metallischen Werkstoffe wird zum Teil Bezug auf das AD 2000-Merkblatt HP 0 Ausgabe 11.2008 (Allgemeine Grundsätze für Auslegung, Herstellung und damit verbundene Prüfungen) und DIN EN 10020 Ausgabe 07.2000 (Begriffsbestimmung für die Einteilung der Stähle) genommen. Die dort genannten Werkstoffgruppen werden zitiert, um die Bezeichnungen (z. B. "austenitscher nichtrostender Stahl) zu präzisieren. Sofern technisch sinnvoll, gelten die Aussagen für alle technisch verfügbaren Varianten der Werkstoffe (z. B. Schmiedevarianten, Walzvarianten und Gussvarianten) mit vergleichbarer Beständigkeit gegen Laugekorrosion.
a) Für drucktragende und produktberührte Komponenten können beliebige, nach dem Stand der Technik geeignete Werkstoffe zur Anwendung kommen wie z. B.:
   - Warmfeste Stähle (z. B. Werkstoffuntergruppe 5.1 bis 5.4 und 6.1 bis 6.4 gemäß AD 2000 HP 0)
   - Austenitische nichtrostende Stähle (z. B. Werkstoffuntergruppe 8.1 bis 8.2 gemäß AD 2000 HP 0)
   - Ferritfreie austenitische nichtrostende Stähle (z. B. Werkstoffuntergruppe 8.1 bis 8.2 gemäß AD 2000 HP 0)
   - Ferritisch-austenitische nichtrostende Stähle (z. B. Werkstoffuntergruppe 10.1 bis 10.2 gemäß AD 2000 HP 0)
   - Nickel und Nickellegierungen (z. B. Werkstoffuntergruppe 41 bis 46 gemäß AD 2000 HP 0)

   Auch Kombinationen der o. g. Werkstoffe können zur Anwendung kommen. Dabei ist die Wahl der Werkstoffe nicht auf die genannte Auswahl beschränkt und umfasst auch korrosionstechnisch gleichwertige oder höherwertige Varianten. Bevorzugt werden Werkstoffe, die sich nach dem Stand der Technik unter Berücksichtigung der Beanspruchungsbedingungen und Gefährdungen durch eine technische Beständigkeit gegen Laugen auszeichnen. Ein Verzicht auf Wärmebehandlungen ist nicht möglich, wenn sich dadurch die technische Beständigkeit gegen Laugen in unzulässiger Weise ändert.
b) Für nicht drucktragende und produktberührte Komponenten können beliebige, nach dem Stand der Technik geeignete Werkstoffe zur Anwendung kommen wie z. B.:
   - Alle unter a) genannten Werkstoffe
   - Unlegierte Stähle (z. B. Werkstoffuntergruppe 1.1 bis 1.2 gemäß AD 2000 HP 0)
   - Unlegierte Stähle und andere legierte Stähle (z. B. gemäß DIN EN 10020)

   Auch Kombinationen der o. g. Werkstoffe können zur Anwendung kommen. Dabei ist die Wahl der Werkstoffe nicht auf die genannte Auswahl beschränkt und umfasst auch korrosionstechnisch gleichwertige oder höherwertige Varianten. Bevorzugt werden Werkstoffe, die sich nach dem Stand der Technik unter Berücksichtigung der Beanspruchungsbedingungen und Gefährdungen durch eine ausreichende Beständigkeit gegen Laugen auszeichnen. Für nicht drucktragende Komponenten können in Abhängigkeit von der Gefährdung gegebenenfalls temporäre Beständigkeiten in Kauf genommen werden. Ein Verzicht auf Wärmebehandlungen ist nicht möglich, wenn sich dadurch die technische Beständigkeit gegen Laugen in unzulässiger Weise ändert.
c) Die Werkstoffeigenschaften werden durch geeignete Fertigungsverfahren verändert, die im Folgenden nach den in der DIN 8580 Ausgabe 09.2003 (Fertigungsverfahren - Begriffe, Einteilung) genannten Bezeichnungen beschrieben sind. Folgende Fertigungsverfahren können für die Verarbeitung der metallischen Werkstoffe z. B. zur Anwendung kommen:
   - Urformen (z. B. Gießen)
   - Umformen (z. B. Kaltumformen und Warmumformen)
   - Trennen (z. B. Spanen mit geometrisch bestimmter Schneide und Spanen mit geometrisch unbestimmter Schneide)
   - Fügen (z. B. Schmelzschweißen)
   - Beschichten (z. B. Beschichten aus dem flüssigen Zustand, Schmelztauchen, Plattieren, Thermisches Spritzen, Sintern, galvanisches Besichten, chemisches Beschichten und Beschichten aus dem gasförmigen und dampfförmigen Zustand)
   - Stoffeigenschaften ändernde Fertigungsverfahren (Verfestigung durch Umformen wie z. B. Schmieden, Walzen, Strahlen; Wärmebehandeln wie z. B. Vergüten, Rekristallisationsglühen, Spannungsarmglühen, Normalisierungsglühen; Thermomechanische Behandlungen wie z. B. die Kombination von Wärmebehandlung und Umformbehandlung; Sintern und Brennen)

   Auch Kombinationen der o. g. Fertigungsverfahren können zur Anwendung kommen. Dabei ist die Wahl der Fertigungsverfahren nicht auf die genannte Auswahl beschränkt. Bevorzugt werden Verfahren, die nach dem Stand der Technik die erforderliche Laugenbeständigkeit der jeweiligen Werkstoffe und Apparate gewährleisten.
d) Folgende Prüfungen an Apparaten und Einbauten sowie insbesondere an deren Schweißverbindungen können beispielsweise zur Anwendung kommen:
   - Magnetpulverprüfung MT
   - Eindringprüfung PT
   - Durchstrahlungsprüfung RT
   - Ultraschallprüfung UT
   - Sichtprüfung VT
   - Härteprüfung HT
   - Legierungsanalyse

Auch Kombinationen der o. g. Prüfverfahren sind möglich. Dabei ist die Wahl der Prüfverfahren nicht auf die genannte Auswahl beschränkt. Es werden Prüfverfahren und Bewertungsgrundlagen bevorzugt, die nach dem Stand der Technik dazu beitragen, die erforderliche Laugenbeständigkeit der jeweiligen Komponenten zu gewährleisten.

### II: Herstellung von Isophoronnitril

Im zweiten Schritt des erfindungsgemäßen Verfahrens wird das erfindungsgemäß hergestellte Isophoron mit HCN unter Bildung von Isophoronnitril (IPN, 3-Cyano-3,5,5-Trimethylcyclohexanon) umgesetzt. Die folgenden Angaben beziehen sich demnach auf diese Schritt II.

Die Basenkatalysierte Umsetzung von Blausäure (HCN) mit alpha, beta-ungesättigten cyclischen (oder acyclischen) Ketonen ist eine bekannte Reaktion (Hydrocyanation of Conjugated Carbonyl Compounds, CHAPTER 3, Wataru Nagata and Mitsuru Yoshioka).

Die Reaktion von Isophoron mit HCN zum IPN lässt sich mit folgender Reaktionsgleichung beschreiben:

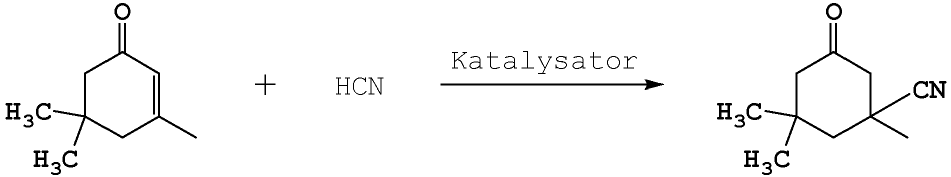

Die Katalysatoren für die Herstellung der beta-Cyanoketone wie z.B. IPN sind generell Basen, die freie Cyanid-Ionen für die 1,4-Additionsreaktion zur Verfügung stellen. Die Umsetzung mit HCN kann entweder in Lösung oder Lösemittelfrei durchgeführt werden.

Als Katalysatoren eignen sich basische Alkali- oder Erdalkaliverbindungen, Oniumverbindungen, organische Basen oder spezielle Verfahren unter Einsatz von Phasentransferkatalysatoren. Die unterschiedlichen Katalysatorsysteme werden nachfolgend näher erläutert:
a) Katalysatoren zur Herstellung von Isophoronnitril aus Isophoron und Blausäure, bestehend aus basischen Alkali- oder Erdalkalimetallverbindungen:
   Entsprechende Beispiele zur Herstellung von Cyanketonen durch Umsetzung von ungesättigten Ketonen basischer Verbindungen mit Blausäure an einem alkalischen, cyanidionen bildenden Katalysator beschreibt DE 1085871. Als geeignete Katalysatoren werden Alkalimetalle und ihre Carbonate; ferner Erdalkalimetalle und Alkali- und Erdalkalialkoholate, - oxyde, -hydroxyde, - peroxyde und -cyanide; tertiäre Amine und quarternäre Ammoniumbasen genannt. Der Katalysator ist in einer Menge von 0,1 bis 20 Gew.-%, bezogen auf das Gesamtgewicht der Reaktionsteilnehmer bezogen, erforderlich. Die Umsetzung erfolgt vorzugsweise bei Temperaturen von 150 bis 225 °C und bei Normaldruck.

   DE 1240521 beschreibt Verfahren zur Herstellung von gamma-Ketocarbon-säurenitrilen aus alpha, beta-ungesättigten Ketonen. Die Herstellung erfolgt, indem hohe Blausäurekonzentrationen und heterogene Katalysatoren (alkalische Katalysatoren auf Trägern, beispielsweise Tonscherben) ohne Lösemittel eingesetzt werden. Als basische Katalysatoren sind z. B. Oxide, Hydroxide, Cyanide und Alkoholate der Alkali- und Erdalkalimetalle genannt. Wesentlich ist, dass die Katalysatoren fest geträgert sind. Es wird bei Temperaturen zwischen 50 und 350 °C synthetisiert.
   Analoge Verfahren sind auch aus DE 1240854 bekannt.
   EP 0433615 beschreibt den Einsatz von Lithiumhydroxid (LiOH) als Katalysator, gemäß EP 0558332 kann auch Lithiumcyanid als Katalysator eingesetzt werden, das zuvor aus LiOH hergestellt wurde.
   Darüber hinaus beschreibt EP01418172 den Einsatz eines Calciumoxides mit einer Oberfläche von > 1,5 m²/g. Die Reaktion findet bevorzugt bei Temperaturen zwischen 150 - 170 °C statt.
b) Katalysatoren zur Herstellung von Isophoronnitril aus Isophoron und Blausäure, bestehend aus Oniumverbindungen oder deren spezieller Verwendung:
   JP 61033157 beschreibt die Herstellung von Isophoronnitril durch Reaktion von Blausäure und Isophoron in Anwesenheit von quaternären Ammonium- oder Phosphoniumhydroxiden als Katalysatoren.

   Gemäß EP 558799 können zur Umsetzung von Isophoron mit HCN auch Onium-Cyanide, beispielsweise Tetraalkylammonium-Cyanid oder Tributylsulfonium-Cyanid als Katalysator eingesetzt werden.
   EP 0671384 betrifft den Einsatz von quaternären Ammoniumsalzen mit Hydrogencarbonat bzw. Alkylcarbonat-Gegenionen.
   Der Einsatz von quaternären Ammonium- oder Phosphoniumsalzen, beispielsweise Ammonium- oder Phosphoniumcyaniden, wird auch in US 5,183,915 oder EP 0502707 beschrieben, wobei im letzteren Falle ein basischer Co-Katalysator (Kalium- oder Natriumcarbonat) eingesetzt wird.
   In der Schrift US 5,011,968 wird die Herstellung von IPN durch Reaktion von Isophoron mit HCN und Tetramethylammoniumhydroxid als Katalysator und anschließender thermischer Zerstörung des Katalysators zu Trimethylamin und Entfernung mit dem Abgas beschrieben.
c) Katalysatoren zur Herstellung von Isophoronnitril aus Isophoron und Blausäure, mittels Phasentransferkatalyse oder unter Verwendung eines zweiphasigen Reaktionssystems:
   EP 28179 beschreibt die Herstellung von IPN aus IP (organische Phase) und Cyaniden (wässrige Phase) in einem 2-Phasensystem mit einem, in katalytischen Mengen eingesetztem Phasenübertragungsmittel. Als Phasentransferkatalysatoren werden quaternäre Ammonium- und Phosphoniumsalze (beispielsweise Tetraethylammoniumbromid oder Dinatriumphosphat) eingesetzt, die in der organischen Phase löslich sind.

   Gemäß US 4,299,775 kann als Katalysator Natrium oder Kaliumcyanid in Gegenwart von Wasser und einem inerten Lösungsmittel sowie eines Onium-Phasentransferkatalysators eingesetzt werden.
   EP 0425806 (DE 69009394T2) beschreibt die Umsetzung von Isophoron mit alkalischen Cyaniden in stöchiometrischen Mengen in homogener Lösung mit einem wässrigen und organischen Lösemittelgemisch, wobei gleichzeitig und allmählich mit anorganischen Säuren neutralisiert wird. Die Phasen entmischen sich während der Reaktion wieder und können auf diese Weise voneinander getrennt werden.
d) Katalysatoren zur Herstellung von Isophoronnitril aus Isophoron und Blausäure, mittels besonderer organischer Basen:
   EP0985659 (analog DE19836474) beschreibt die Verwendung von 1,3-Dimethyl-imidazolium-4-carboxylat als Katalysator.

   Diazo-bicycloalkene sind ebenfalls als Katalysatoren für die Herstellung von IPN durch Reaktion von Blausäure und Isophoron geeignet (JP 61033158).
   In JP04253948 wird Isophoron zu IPN mit HCN in Gegenwart von Guanidin (0,01 - 0,5 Mol bezogen auf 1 Mol Isophoron) als Base umgesetzt.
e) Katalysatoren zur Herstellung von Isophoronnitril aus Isophoron und Blausäure, mittels Einsatzes besonderer Lösemittel:
   In JP57116038 wird Isophoron zu IPN mit HCN in Gegenwart eines basischen Katalysators und einem Glykol (beispielsweise Ethylenglykol) umgesetzt.

JP04164057 beschreibt die Herstellung von IPN durch äquimolare Umsetzung von IP mit HCN unter Basenkatalyse in 1,3-Dimethyl-2-imidazolidinon als Lösemittel.

JP04112862 beinhaltet die Herstellung von IPN durch Reaktion von Isophoron mit HCN, alkalischen Katalysatoren und DMSO (Dimethylsulfoxid) und/oder DMF (Dimethylformamid als Lösemittel.

Von den oben genannten Katalysatoren werden in dem erfindungsgemäßen Verfahrenschritt vorzugsweise jene aus der Gruppe der basischen Alkali- oder Erdalkalimetallverbindungen eingesetzt.

Die Umsetzung kann dabei erfindungsgemäß sowohl homogen oder heterogen basenkatalysiert durchgeführt werden. Bei der homogenen Katalyse liegen Katalysator und Reaktanden in der gleichen Phase vor. Typisch ist ein in einem Lösungsmittel gelöstes Metallsalz (z. B. 25 Gew.-%ige Natriummethanolatlösung, gelöst in Methanol), an dem die Reaktanden reagieren.

In der heterogenen Katalyse liegen Katalysator und Reaktanden in unterschiedlichen Phasen vor. Meist ist ein fester Katalysator (z. B. Metalloxid) in flüssiger Phase vorliegenden Reaktanden ausgesetzt, also wenn zwischen dem Katalysator und dem Reaktionsgemisch eine Phasengrenze vorhanden ist. Der Katalysator ist dabei in der Regel ein Feststoff, während die Reaktanden gasförmig und/oder flüssig sind. Bei heterogen katalysierten Prozessen spielen sich die entscheidenden Vorgänge an der Oberfläche des Festkörpers ab.

Generell eignen sich in dem erfindungsgemäßen Verfahrensschritt sowohl die homogene als auch die heterogene Basenkatalyse. Insbesondere handelt es sich bei den Katalysatoren um Alkalimetalle und ihre Carbonate; ferner Erdalkalimetalle und Alkali- und Erdalkalialkoholate, -oxyde, -hydroxyde, -peroxyde und -cyanide.
In dem erfindungsgemäßen Verfahrensschritt wird vorzugsweise eine homogene Basenkatalyse eingesetzt. Hierzu werden ganz besonders bevorzugt Alkalialkoholate, insbesondere Natriummethanolat, als Katalysatoren eingesetzt.

Die eingesetzte Blausäure kann in reiner Form aber auch als Gemisch mit Stabilisatoren eingesetzt werden. Als Stabilisatoren eignen sich alle dem Fachmann für diesen Zweck bekannten Verbindungen, beispielsweise Phosphorsäure, Schwefeldioxid und Oxalsäure. Bevorzugt werden Phosphorsäure, Schwefeldioxid oder Gemische hieraus als Stabilisatoren für die Blausäure eingesetzt. Der Anteil der Stabilisatoren liegt üblicherweise im Bereich von 0,1 bis 2 Gew.-%, bezogen auf die eingesetzte Blausäure, vorzugsweise im Bereich von 0,5 bis 1 Gew.-%.

Die Umsetzung kann in Gegenwart oder Abwesenheit von inerten Lösungsmitteln vorgenommen werden. Besonders bevorzugt verwendet man Isophoron im molaren Überschuss, bezogen auf die eingesetzte Blausäure und setzt kein externes Lösungsmittel zu.

Generell wird das erfindungsgemäße Verfahren derart durchgeführt, dass ein Überschuss an Isophoron eingesetzt wird, da so eine höhere Selektivität zu Isophoronnitril erzielt wird. Im Allgemeinen beträgt das molare Verhältnis Isophoron/HCN >1:1, in der Regel 19:1 bis 1,5:1, vorzugsweise 3:1 bis 1,5:1.

Die Katalysatorkonzentration, bezogen auf die eingesetzte Isophoronmenge, liegt im Bereich von 0,03 bis 20 Gew.-%, vorzugsweise im Bereich von 0,03 bis 1 Gew.-%.

Die Gesamtmenge des Isophorons kann vorgelegt und auf die gewünschte Reaktionstemperatur gebracht werden, bevor in Anwesenheit des Katalysators das HCN zugegeben wird.

Die HCN Zudosierung erfolgt vorzugsweise so, dass eine homogene Verteilung der Edukte (Isophoron und HCN) gewährleistet wird. Die HCN Zudosierung geschieht auf übliche, dem Fachmann bekannte Weise, z. B. über Statikmischer, Pumpen oder Verdüsung.

Die Reaktionstemperaturen liegen üblicherweise im Bereich von 130 bis 225 °C, bevorzugt im Bereich von 135 bis 195 °C und ganz besonders bevorzugt im Bereich von 140 bis 175 °C.

Die Reaktion wird bei Drücken von 0,01 bis 10 bar, bevorzugt 1 bis 5 bar, besonders bevorzugt bei Normaldruck (Atmosphärendruck) durchgeführt.

Innerhalb der vorstehend dargelegten Reaktionsparameter wird das HCN so zudosiert, dass eine ausreichend niedrige Konzentration von HCN resultiert und somit eine hohe Selektivität sowie ein hoher Umsatz zu Isophoronnitril erzielt werden kann. Die Selektivität hinsichtlich IPN sollte > 95 %, bevorzugt > 98 %, besonders bevorzugt > 99 %, liegen.

Dabei darf nur eine geringe Polymerisation des HCN auftreten, da hierdurch der Umsatz und die Selektivität negativ beeinflusst würden.

Vorzugsweise werden die Konzentrationen an nicht umgesetztem, freiem HCN und die Gesamtkonzentration an Cyanidionen (Summe aus freiem HCN und als Cyanhydrine von Isophoron und Isophoronnitril gebundenem Cyanid) bestimmt und die Reaktionsbedingungen so angepasst, dass eine optimierte Selektivität hinsichtlich IPN erhalten wird. Die Bestimmung der genannten Cyanidionen-Konzentrationen erfolgt vorzugsweise durch Titration.

Das erfindungsgemäße Verfahren kann in bevorzugter Weise kontinuierlich, diskontinuierlich oder semikontinuierlich durchgeführt werden, insbesondere kontinuierlich.

In bevorzugter Ausführung wird die Reaktionsdurchführung, je nach eingesetztem Katalysator, in einem Rührkessel, einer Rührkesselkaskade, einem Kreislaufreaktor, einem Strömungsrohr, einem oder mehreren Festbettreaktoren oder einer Kolonne durchgeführt.

Die Aufarbeitung des Reaktionsgemisches nach beendeter Reaktion geschieht auf übliche, dem Fachmann bekannte Weise.

Gemäß JP 06065182 kann nach Durchführung der Reaktion von HCN mit IP der alkalische Katalysator neutralisiert und das Reaktionsgemisch direkt in der Destillation aufgearbeitet werden.

Alternativ kann gemäß JP 06065183 das alkalische Reaktionsgemisch mit einem inerten Lösemittel vermischt und in einem Dünnschichtverdampfer aufgearbeitet werden. Dabei werden der Katalysator, die Hochsieder und das Lösemittel abgetrennt. Das erhaltene Isophoronnitril wird dann in einer weiteren Kolonne aufgereinigt.

Die Abtrennung des Katalysators kann beispielsweise nach den in DE 1085871 oder EP 0433615 beschrieben Verfahren erfolgen.

Vorzugsweise wird überschüssiges Isophoron durch Destillation abgetrennt und vorteilhafterweise erneut eingesetzt. Dazu wird das abgetrennte Isophoron mit frischem Isophoron vermischt und in den Reaktionsreaktor zurückgeführt. Das gebildete Isophoronnitril wird durch destillative Aufarbeitung von möglichen Nebenkomponenten abgetrennt.

### III: Herstellung von 3-Aminomethyl-3,5,5-trimethylcyclohexylamin

Im dritten Schritt des erfindungsgemäßen Verfahrens wird das erfindungsgemäß hergestellte Isophoronnitril mittels katalytischer Hydrierung und/oder katalytischer reduktiver Aminierung zu Isophorondiamin umgesetzt. Die folgenden Angaben beziehen sich demnach auf diese Schritt III.

Gegenstand der Erfindung gemäß Schritt III. ist ein Verfahren zur Herstellung von Isophorondiamin, mittels katalytischer Hydrierung und/oder katalytischer reduktiver Aminierung von Isophoronnitril (IPN), in Gegenwart von Ammoniak, Wasserstoff und mindestens eines Katalysators sowie gegebenenfalls eines Lösungsmittels oder Lösungsmittelgemisches, wobei die Cyanidionenkonzentration im Reaktionsgemisch, das der Hydrierung zugeführt wird von 200 ppmw bis 5000 ppmw beträgt, bezogen auf das eingesetzte Isophoronnitril.

Die Einstellung der Cyanidionenkonzentration von 200 ppmw bis 5000 ppmw, bevorzugt bis 3000 kann durch verschiedene Maßnahmen erreicht werden, beispielsweise durch gezielte Zudosierung von HCN oder Cyanidsalzen wie KCN oder auch durch den Einsatz geeigneter IPN-Qualitäten. In dem erfindungsgemäßen Verfahren wird die Einstellung der Cyanidionenkonzentration bevorzugt dadurch erreicht, dass eine gezielte Rückspaltung des IPN in der Iminierungsstufe hervorgerufen wird. Dies kann erfindungsgemäß entgegen der Lehre von EP 394 967 A1 durch eine Erhöhung der Temperatur in der Iminierungsstufe um 5-50 K, bevorzugt 7-30 K, besonders bevorzugt 10-20 K oberhalb der Temperatur, die, abhängig vom Einsatz eines Iminierungskatalysators, notwendig ist, um einen Umsatz von IPN zu IPNI von mindesten 80 % in der Iminierungsstufe zu erreichen.

Es ist möglich, das erfindungsgemäße Verfahren gemäß Schritt III. in einer Stufe oder in mehreren Stufen durchzuführen.

Wird das Verfahren in einer Stufe durchgeführt, wird Isophoronnitril direkt in Anwesenheit von Ammoniak, Wasserstoff, einem Hydrierkatalysator und gegebenenfalls weiteren Zusätzen und in An- oder Abwesenheit von organischen Lösungsmitteln aminierend hydriert.

Der Begriff "in mehreren Stufen" bedeutet, dass zunächst in einem separaten Reaktor oder Reaktorabschnitt Isophoronnitril zunächst ganz oder teilweise in Isophoronnitrilimin umgewandelt wird, und dieses Isophoronnitrilimin als Reinsubstanz oder im Gemisch mit anderen Komponenten unter Anwesenheit von mindestens Ammoniak aminierend hydriert wird.

Eine bevorzugte Ausführungsform des erfindungsgemäßen Verfahrens zur Herstellung von IPDA gemäß Schritt III. ist ein zweistufiger Prozess: In der ersten Stufe wird zumindest ein Teil des eingesetzten IPN bei An- oder Abwesenheit eines Iminierungskatalysators und/oder von Lösungsmitteln durch Reaktion mit Ammoniak in Isophoronnitrilimin umgewandelt. Der Umsatz von IPN zu IPNI sollte nach der Iminierung größer 80 %, bevorzugt größer 90 %, besonders bevorzugt größer 95 % betragen.
In der zweiten Stufe wird das Reaktionsprodukt der ersten Stufe, so wie es anfällt oder nach einer Weiterbehandlung und/oder Zugabe weiteren Ammoniaks, unter Anwesenheit von mindestens Ammoniak und Wasserstoff und in An- oder Abwesenheit eines organischen Lösungsmittel bei einer Temperatur von 20 bis 150 °C, bevorzugt 40 bis 130 °C, und einem Druck von 0,3 bis 50 MPa, bevorzugt 5 bis 30 MPa, an Hydrierkatalysatoren aminierend hydriert.

In einer weiteren bevorzugten Ausführungsform gemäß Schritt III. erfolgt die Umsetzung von IPN zu IPDA in drei voneinander getrennten Reaktionsräumen. In dem ersten Reaktionsraum erfolgt die Umsetzung von IPN zu Isophoronnitrilimin mit überschüssigem Ammoniak an Iminierungskatalysatoren bei Temperaturen zwischen 20 und 150 °C und Drücken zwischen 5 und 30 MPa. Im zweiten Reaktionsraum werden die entstandenen Reaktionsprodukte mit Wasserstoff in Gegenwart von überschüssigem Ammoniak an Hydrierkatalysatoren bei Temperaturen zwischen 20 und 130 °C und Drücken von 5 bis 30 MPa hydriert. Im dritten Reaktionsraum werden die entstanden Reaktionsprodukte an den erfindungsgemäß einzusetzenden Katalysatoren bei Temperaturen zwischen 100 und 160 °C und Drücken von 5 bis 30 MPa hydriert.

Um die Gleichgewichtseinstellung der Iminierungsreaktion zu beschleunigen ist es zweckmäßig, einen Iminierungskatalysator zu verwenden. Hierzu können die nach dem Stand der Technik bekannten Iminierungskatalysatoren verwendet werden. Geeignete Katalysatoren sind beispielsweise anorganische oder organische Ionenaustauscher (siehe EP 042 119), geträgerte Heteropolysäuren (siehe DE 44 26 472), acide Metalloxide, insbesondere Aluminiumoxid und Titandioxid (siehe EP 449 089) Sulfonsäuregruppen enthaltende Organopolysiloxane (DE 196 27 265.3) und saure Zeolithe sowie Aktivkohle (EP 061 137). Bei Verwendung eines Iminierungskatalysators kann die Reaktionstemperatur zwischen 10 und 150 °C, vorzugsweise zwischen 30 und 130 °C und ganz besonders bevorzugt zwischen 40 und 100 °C liegen. Der Druck liegt zwischen dem Eigendruck der Mischung und 50 MPa. Bevorzugt wird die Iminierungsreaktion bei dem Druck durchgeführt, bei dem auch die anschließende reduktive Aminierung durchgeführt wird.

Obwohl die Iminierung von Isophoronnitril mit flüssigem Ammoniak bevorzugt ohne Zugabe weiterer Lösungsmittel durchgeführt wird, kann auch in Anwesenheit zusätzlicher Lösungsmittel gearbeitet werden. Geeignet sind einwertige Alkohole mit 1 bis 4 C-Atomen, insbesondere Methanol sowie Ether, besonders THF, MTBE und Dioxan.

In der Iminierungsstufe werden pro Mol eingesetztem IPN zwischen 1 und 500 Mol, bevorzugt 5 und 200 Mol, besonders bevorzugt zwischen 5 und 100 Mol Ammoniak eingesetzt. Typische Katalysatorbelastungen liegen im Bereich von 0,01 bis 10 kg IPN je kg Katalysator und Stunde, bevorzugt 0,5 bis 10 und besonders bevorzugt 0,5 bis 5 kg IPN je kg Katalysator und Stunde.

Bei der Iminierung in Gegenwart eines Iminierungskatalysators kann der Katalysator in Form eines Suspensionskatalysators oder Festbettkatalysators vorliegen. Vorteilhaft ist die Verwendung von Festbettkatalysatoren. In einer besonders bevorzugten Ausführungsform werden IPN und Ammoniak kontinuierlich von unten nach oben durch ein mit Iminierungskatalysator gefülltes Reaktionsrohr geleitet.

Die Hydrierung erfolgt üblicherweise bei Temperaturen zwischen 20 und 150 °C, bevorzugt 40 und 130 °C, und Drücken von 0,3 bis 50 MPa, bevorzugt 5 bis 30 MPa. Es ist auch möglich, die Hydrierung in Gegenwart der bereits bei der Iminierungsstufe genannten Lösungsmittel durchzuführen. Der wesentliche Vorteil bei Verwendung eines Lösungsmittels liegt darin, dass die Hydrierung bei niedrigeren Drücken zwischen 0,3 und 10 MPa durchgeführt werden kann.

Der für die Hydrierung erforderliche Wasserstoff kann dem Reaktor entweder im Überschuss, beispielsweise mit bis zu 10000 Moläquivalenten, zugeführt werden, oder nur in einer solchen Menge, dass der durch Reaktion verbrauchte Wasserstoff sowie der Teil des Wasserstoffs, der eingelöst im Produktstrom den Reaktor verlässt, nachgeführt wird. Bei kontinuierlicher Fahrweise kann der Wasserstoff im Gleich- oder Gegenstrom zugeführt werden.

In einer bevorzugten Ausführungsform erfolgt die Hydrierung in flüssigem Ammoniak als Lösungsmittel. Pro Mol IPN werden zwischen 1 und 500 Mol, bevorzugt 5 und 200 Mol, besonders bevorzugt zwischen 5 und 100 Mol Ammoniak verwendet. Zweckmäßigerweise verwendet man mindestens die Ammoniakmenge, die bei der vorgelagerten Iminierung eingestellt wurde. Der Ammoniakanteil kann aber auch vor der Hydrierung durch Zugabe von zusätzlichem Ammoniak auf den gewünschten Wert erhöht werden.

Als Katalysatoren können prinzipiell alle Katalysatoren eingesetzt werden, die die Hydrierung von Nitril- und/oder Imingruppen mit Wasserstoff katalysieren. Besonders geeignet sind Nickel-, Kupfer-, Eisen-, Palladium-, Rhodium-, Ruthenium- und Cobaltkatalysatoren, ganz besonders Ruthenium- und Cobaltkatalysatoren. Zur Erhöhung der Aktivität, Selektivität und/oder Standzeit können die Katalysatoren zusätzlich Dotiermetalle oder andere Modifizierungsmittel enthalten. Typische Dotiermetalle sind z. B. Mo, Fe, Ag, Cr, Ni, V, Ga, In, Bi, Ti, Zr und Mn sowie die seltenen Erden. Typische Modifizierungsmittel sind z. B. solche, mit denen die Säure-Base-Eigenschaften der Katalysatoren beeinflusst werden können, bevorzugt Alkali- und Erdalkalimetalle bzw. deren Verbindungen, bevorzugt Mg- und Ca-Verbindungen, sowie Phosphorsäure oder Schwefelsäure sowie deren Verbindungen.

Die Katalysatoren können in Form von Pulvern oder Formkörpern, wie z. B. Extrudaten oder gepressten Pulvern, eingesetzt werden. Es können Vollkontakte, Raney-Typ-Katalysatoren oder Trägerkatalysatoren zur Anwendung kommen. Bevorzugt sind Raney-Typ- und Trägerkatalysatoren. Geeignete Trägermaterialien sind z. B. Siliciumdioxid, Aluminiumoxid, Alumosilikate, Titandioxid, Zirkoniumdioxid, Kieselgur, Aluminium-Silicium-Mischoxide, Magnesiumoxid und Aktivkohle. Das Aktivmetall kann in dem Fachmann bekannter Weise auf das Trägermaterial aufgebracht werden, wie z. B. durch Imprägnierung, Aufsprühen oder Fällung. Je nach Art der Katalysatorherstellung sind weitere, dem Fachmann bekannte, Präparationsschritte notwendig, wie z. B. Trocknung, Calcinierung, Formgebung und Aktivierung. Zur Formgebung können optional weitere Hilfsstoffe wie z. B. Graphit oder Magnesiumstearat zugesetzt werden. Das erforderliche Volumen der einzusetzenden Hydrierkatalysatoren richtet sich nach dem vom Betriebsdruck, der Temperatur, der Konzentration und der Katalysatoraktivität abhängigen LHSV-Wert, (liquid hourly space velocity) der eingehalten werden muss, um eine möglichst vollständige Hydrierung des eingesetzten IPN zu gewährleisten. Üblicherweise liegt der LHSV-Wert bei der Verwendung des bevorzugt einzusetzenden Gemisches aus IPN, Ammoniak und Wasserstoff zwischen 0,5 und 4 Liter IPN/Ammoniak-Mischung pro Liter Katalysator und Stunde, bevorzugt zwischen 1 und 3 I_{Lsg} I_{Kat}⁻¹ h⁻¹.

Es ist bevorzugt, dass die einzusetzenden Hydrierkatalysatoren vor ihrem Einsatz in der Hydrierung zunächst mit Ammoniak konditioniert werden. Dazu werden die Katalysatoren mit Ammoniak oder mit Mischungen aus Ammoniak und einem oder mehrerer Lösungsmittel in Kontakt gebracht. Bevorzugt erfolgt die Konditionierung nach Einbau der Katalysatoren im Hydrierreaktor, sie kann aber auch vor Einbau der Katalysatoren erfolgen. Zur Konditionierung werden zwischen 0,2 und 3, bevorzugt 0,5 und 2 m³ Ammoniak pro m³ Katalysator und Stunde eingesetzt. Üblicherweise wird bei Temperaturen zwischen 20 und 150 °C, bevorzugt 40 bis 130 °C, gearbeitet. Besonders bevorzugt wird eine Temperaturrampe durchfahren, bei der der Katalysator beginnend bei mäßig erhöhter Temperatur, bevorzugt zwischen 20 und 50 °C, langsam bis auf die später für die Hydrierung gewünschte Reaktionstemperatur, bevorzugt 20 bis 150 °C, aufgeheizt wird. Die Konditionierung wird bevorzugt in Gegenwart von Wasserstoff durchgeführt, wobei der Partialdruck des verwendeten Wasserstoffs im Reaktor den Bereich von 0,1 bis 50 MPa, bevorzugt 5 bis 40 MPa, besonders bevorzugt 10 bis 30 MPa umfasst. Die Zeitspanne der Konditionierung ist abhängig von der verwendeten Ammoniakmenge und liegt bevorzugt zwischen 1 und 48 h, besonders bevorzugt zwischen 12 und 24 h.

In dem bevorzugten zweistufigen Prozess wird das Isophoronnitrilimin enthaltende Gemisch mit Hilfe eines geformten Hydrierkatalysators in der zweiten Stufe hydriert. Das der Hydrierstufe zugeführte Gemisch kann unmittelbar jenes sein, welches bei der Iminierung von IPN mit Ammoniak in der der ersten Stufe anfällt, oder wie es nach Zugabe oder Entfernung von Komponenten, wie z. B. Ammoniak, organischen Lösungsmitteln, Basen, CoKatalysatoren, Cyanidsalze, Blausäure und/oder Wasser, erhalten wird. Bevorzugt wird die Hydrierung kontinuierlich in Festbettreaktoren durchgeführt, die in Riesel- oder Sumpffahrweise betrieben werden können. Geeignete Reaktortypen sind z. B. Schachtöfen, Hordenreaktoren oder Rohrbündelreaktoren. Es ist auch möglich, für die Hydrierung mehrere Festbettreaktoren hintereinander zu schalten, wobei jeder der Reaktoren wahlweise in Rieselbett- und Sumpffahrweise betrieben wird.

Außer den zuvor genannten Bestandteilen des der Iminierungsstufe zuzuführenden Gemisches kann dieses zusätzlich höher oder niedriger als IPDA siedende Fraktionen aus der destillativen Aufarbeitung des aus dem Rieselbettreaktor abgezogenen Reaktionsgemisches enthalten. Derartige Fraktionen können außer Resten an IPDA auch solche Nebenprodukte enthalten, aus denen sich unter Reaktionsbedingungen erneut IPDA bildet. Besonders vorteilhaft ist es, die oberhalb IPDA siedende Fraktion, welche außer Resten an IPDA 2-Aza-4,6,6-trimethylbicyclo[3.2.1]-octan als Hauptprodukt enthält, zurückzuführen. Ebenfalls besonders vorteilhaft ist es, nicht vollständig umgesetztes IPN, insbesondere Isophoronaminonitril enthaltende Fraktionen, zurückzuführen. Die Rückführungen können auch, falls dieses gewünscht ist, unmittelbar dem der Hydrierstufe zuzuführenden Reaktionsgemisch zugesetzt werden.

Bei der Hydrierung von IPN bzw. Isophoronnitrilimin können zwei unterschiedliche Stereoisomere gebildet werden. Durch die Wahl eines Temperaturprofils im Hydrierschritt lässt sich das Isomerenverhältnis beeinflussen. Es ist z. B. möglich, ein IPN oder Isophoronnitrilimin enthaltendes Gemisch zunächst bei einer Temperatur zwischen 20 und 90 °C teilweise zu hydrieren, und anschließend die Reaktion in einem zweiten Schritt in einem Temperaturbereich zwischen 90 und 150 °C zu vervollständigen. Durch die Einhaltung relativ niedriger Reaktionstemperaturen im 1. Schritt kann die Selektivität zu Gunsten des cis-Isomeren verschoben werden. Die Einhaltung relativ niedriger Reaktionstemperaturen zu Beginn der Reaktion hat zudem den Vorteil, dass das thermisch labile Isophoronnitrilimin besonders schonend hydriert wird und dadurch Nebenreaktionen unterbunden werden. Das intermediär gebildete Isophoronaminonitril ist thermisch deutlich stabiler und kann daher bei höheren Temperaturen hydriert werden, ohne dass weitere Nebenreaktionen befürchtet werden müssen. Zu den unerwünschten Nebenreaktionen zählt auch die Abspaltung von HCN. Nach dem erfindungsgemäßen Verfahren wirkt sich eine gewisse Cyanidionenkonzentration positiv auf die Selektivität der Hydrierstufe aus. Dieser Effekt tritt verstärkt dann zu Tage, wenn die Cyanidionen schon von Anfang an in der Hydrierstufe vorliegen und nicht erst während der Hydrierung entstehen. Daher ist eine Abspaltung von HCN während der Hydrierstufe zu verhindern.

Die Realisierung des gewünschten Temperaturprofils kann beispielsweise durch die Hintereinanderschaltung von zwei oder mehr getrennt voneinander beheizbaren Reaktoren erfolgen. Es ist aber auch möglich, ein ansteigendes Temperaturprofil in nur einem Hydrierreaktor zu realisieren. Besonders bevorzugt erfolgt die Durchführung der Hydrierreaktion in einem adiabatisch betriebenen Rieselbettreaktor, bei dem das Reaktionsgemisch dem Reaktor bei Temperaturen zwischen 20 und 90 °C zugeführt wird, und aufgrund der auftretenden und durch das Reaktionsgemisch aufgenommenen Reaktionswärme zwischen 90 und 150 °C wieder verlässt.

Das die Hydrierung verlassende Reaktionsgemisch wird mit den üblichen Methoden weiter aufgereinigt, um ein IPDA mit der gewünschten Qualität zu erhalten. Dabei können alle gängigen Trennmethoden wie z. B. Destillation, Entspannungsverdampfung, Kristallisation, Extraktion, Sorption, Permeation, Phasentrennung oder Kombinationen aus den genannten zum Einsatz kommen. Die Aufreinigung kann kontinuierlich, absatzweise, ein- oder mehrstufig, im Vakuum oder unter Druck durchgeführt werden. Mögliche Komponenten, die beispielsweise bei der weiteren Aufreinigung abgetrennt werden, sind Wasserstoff, Ammoniak, Wasser sowie die bei der Herstellung von IPDA aus IPN anfallenden Nebenprodukte wie z. B. hydrierte HCN-Eliminierungsprodukte oder Verunreinigungen des IPN, methylierte Nebenprodukte und/oder unvollständig hydrierte Zwischenprodukte.

Bevorzugt wird die Aufreinigung durch Destillation unter Druck und/oder im Vakuum in mehreren Schritten erreicht. Hierfür lassen sich beliebige Destillationskolonnen mit und ohne Einbauten wie z. B. Dephlegmatoren, Trennwänden, ungeordnete Einbauten oder Schüttungen, geordnete Einbauten oder Packungen, Böden mit oder ohne Zwangsführung verwenden.

In einem ersten Schritt werden insbesondere Wasserstoff, Inertgase, Ammoniak, leicht siedende Verunreinigungen und ggf. auch Wasser in einer oder mehreren Destillationskolonnen ganz oder teilweise abgetrennt. Dabei erfolgt die Abtrennung bevorzugt bei einem Druck, der kleiner ist als im Reaktionsschritt. Erfolgt die Abtrennung in mehreren Destillationsschritten, so ist es vorteilhaft, das der Druck stufenweise abgesenkt wird. Ganz besonders bevorzugt erfolgt die Abtrennung oberhalb 1 bar und mit Sumpftemperaturen von 0 - 200 °C. Der Einsatz eines Stripgases zur Entfernung von leicht siedenden Verunreinigungen kann vorteilhaft sein. Insbesondere Ammoniak und Wasserstoff und Anteile der leicht siedenden Verunreinigungen können ganz oder zum Teil in den Prozess (Reaktion) zurückgeführt werden. Die leicht siedenden Verunreinigungen und gegebenenfalls Anteile von Wasserstoff und Ammoniak werden der thermischen Verwertung zugeführt.

In einem zweiten Schritt werden weitere leicht siedende Verunreinigungen, Wasser und schwer siedende Verunreinigungen ganz oder teilweise abgetrennt. Dies kann in einer oder mehreren Destillationskolonnen erfolgen. Dabei kann Wasser zusammen mit organischen, leicht siedenden Verunreinigungen und gegebenenfalls Anteilen an IPDA über Kopf der Kolonne abdestilliert werden und nach Kondensation in eine wässrige und eine organische Phase getrennt werden. Hierbei kann die organische Phase teilweise als Rückfluss in die Kolonne zurückgeführt werden. Wird der zweite Schritt der Destillation in einer einzigen Kolonne durchgeführt (z. B. einer Trennwandkolonne), so wird das IPDA mit der gewünschten Reinheit über einen Seitenstrom entnommen, während die schwer siedenden Verunreinigungen im Sumpf der Kolonne anfallen. Wird die Trennung aber zwei- oder mehrstufig durchgeführt, so wird das IPDA am Kopf einer Kolonne erhalten. Die Abtrennung der leicht und schwer siedenden Verunreinigungen und von Wasser erfolgt bevorzugt im Vakuum zwischen 100 Pa und 0,0999 MPa und Sumpftemperaturen von 50 - 300 °C. Sämtliche Nebenkomponenten können der thermischen Verwertung zugeführt werden.

Die vorliegende Erfindung gemäß Schritt III. ist also insbesondere dadurch gekennzeichnet, dass entgegen dem bisherigen Stand der Technik eine Maximierung der Selektivität der reduktiven Aminierung von IPN zu IPDA nicht durch die explizite Minimierung der Cyanidionenkonzentration erfolgt. Vielmehr ist eine Mindestkonzentration von 200 ppmw bezogen auf die Masse an eingesetztem IPN förderlich für die Selektivität der Hydrierung des IPNI zum IPDA. Insbesondere wird die Bildung der bicyclischen Verbindung 2-Aza-4,6,6-trimethylbicyclo[3.2.1]-octan, einem Hauptnebenprodukt bei der reduktiven Aminierung von IPN zu IPDA, das durch den intramolekularen nukleophilen Angriff der Amingruppe des IPAN an das C-Atom der Nitrilgruppe gebildet wird, signifikant verringert.

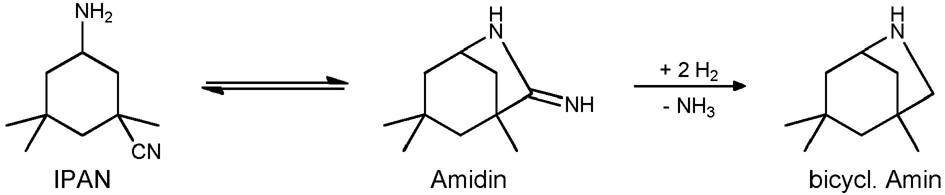

Die Beispiele zu III, 1 und 2 zeigen zwei vergleichbare Versuchseinstellungen, die sich einzig durch die Cyanidionenkonzentration im Feed unterscheiden. Es wird deutlich, dass durch die Zudosierung einer Menge Cyanid, die 1000 ppmw HCN entspricht, die Menge an gebildetem bicyclischem Amin von 4,13 % auf 2,03 % verringert wird. Das Zwischenprodukt Amidin wird von 1,40 % auf 1,00 % abgesenkt. Da keine verringerte Aktivität festgestellt werden konnte (Umsatz von IPN und IPAN konstant), steigt die Ausbeute an IPDA im Rohprodukt auf von 93,23 % auf 95,69 %.
Neben dem positiven Einfluss auf die Selektivität besitzen die Cyanidionen aber parallel die in der Literatur beschriebene vergiftende Wirkung auf Hydrierkatalysatoren. Daher ist eine übermäßige Erhöhung der Cyanidionenkonzentration nicht zielführend, da sonst die desaktivierende Wirkung überhand nimmt. Bevorzugt ist eine Konzentration bezogen auf das eingesetzte IPN von maximal 3000 ppmw. Eine deutlich höhere Cyanidkonzentration von 5000 ppmw bezogen auf das eingesetzte IPN, sorgt nach wie vor für verringerte Nebenproduktkonzentrationen (bicyclisches Amin 2,36 %, Amidin 1,03 %), allerdings steigt der Anteil an nicht umgesetztem IPAN von 0,63 % auf 1,15 %, wodurch sich die Gesamtausbeute an IPDA mit 94,62 % um einen Prozentpunkt verringert. Dies ist in Beispiel 3 zu III. verdeutlicht.

### Beispiele

### Zu I.

### Beispiel erfindungsgemäß:

Ein Roh-Isophoron-Gemisch bestehend aus Isophoron, leichtersiedenden Komponenten, höhersiedenden Komponenten sowie Wasser und Katalysator, welches auf eine der oben beschriebenen Verfahrensweisen erzeugt wurde, wird dem Hydrolyseapparat entnommen, auf ca. 40 - 60 °C abgekühlt und einer Phasentrennung unterzogen. Das Phasenverhältnis beträgt 1 Teil organische Phase, 4 Teile wässrige Phase. In der wässrigen Phase sind danach noch 1 Gew.- % Isophoron enthalten, dies entspricht etwa 4 Gew.-% der organischen Phase. Bei der nachfolgenden Abwasserdestillation wird der Isophoronanteil nahezu vollständig und das Wasser zu 75 Gew.-% verdampft und in die Hydrolyseapparatur geleitet.
Bei der Abkühlung des Sumpfes der Abwasserkolonne durch Entspannungsverdampfung werden weitere 25 Gew.-% des Wassers zurückgewonnen.

Rechnung für 1 Tonne Isophoronproduktion: 4 Gew.-% (ca. 40 kg) Isophoron Mehrproduktion, Minimierung der Abwassermenge, organische Belastung des Abwassers gering.

### Vergleich, nicht erfindungsgemäß:

Vergleich mit dem herkömmlichen Verfahren: ca. 4 Gew.-% Isophoronverlust über das Abwasser, bezogen auf 1 Tonne Isophoronproduktion. Der Mehrbedarf an Wasser entspricht der 5-fachen Menge, bezogen auf 1 Tonne Isophoronproduktion. Ebenso ist die Abwassermenge 5 mal höher, bezogen auf 1 Tonne Isophoronproduktion.

### Zu II.

### Allgemeine Angaben

Die Versuche zur Bildung von Isophoronnitril aus Isophoron und Blausäure wurden isotherm und diskontinuierlich bzw. halbkontinuierlich in einer Rührkesselapparatur mit Tropftrichter und Rückflusskühler durchgeführt. Die Durchführung eines Standardversuches erfolgte derart, dass die Hauptmenge Isophoron und eine Teilmenge der Blausäure (stabilisiert mit 0,5 - 1,0 % Phosphorsäure) im Reaktor vorgelegt und auf die gewünschte Reaktionstemperatur gebracht wurden. Die Blausäure wurde in einem Gemisch mit Isophoron über einen Tropftrichter zugegeben. Der Zeitpunkt t = 0 wurde durch die Zugabe des Katalysators festgelegt. Kurz vor der Zugabe des Katalysators wurde die Zusammensetzung des Reaktionsgemisches durch eine Probenahme und Gaschromatographische Analyse bestimmt, um mögliche Änderungen während der Aufheizphase zu berücksichtigen. Nach Zugabe des Katalysators wurde die Restmenge Blausäure/Isophoron kontinuierlich zugetropft. Die Zudosierung der Blausäure darf nicht zu schnell erfolgen, da es sonst durch die gebildeten Cyanid-Ionen zu einer Polymerisation der Blausäure kommt. Es entsteht dabei ein brauner polymerer Feststoff, der abgetrennt werden muss. Die Probennahmen erfolgten zu vorgegebenen Zeitpunkten mit einer Spritze. Die Proben wurden ausgewogen und analysiert (Bestimmung des Cyanidgehaltes durch Titration und GC-Bestimmung). Diese Bestimmungen der Reaktionsmischung können durch dem Fachmann bekannte Verfahren erfolgen. Bevorzugt wird das überschüssige Isophoron durch eine Destillation abgetrennt und wieder dem Prozess zugeführt. Die Aufarbeitung des Reaktionsgemisches nach beendeter Reaktion geschieht auf übliche, dem Fachmann bekannte Weise. Vorzugsweise wird überschüssiges Isophoron durch Destillation abgetrennt und vorteilhafterweise erneut eingesetzt. Danach wird das gebildete Isophoronnitril, vorzugsweise ebenfalls destillativ, von Nebenkomponenten getrennt.

Gaschromatographische Bestimmung der Reaktionsprodukte:
Gerät: HP3 / Agilent GC 6890
Tennsäule: HP-5 Agilent (19091J-433) 30m x 250µm x 0.25µm nominal
Const Flow: 0,9 ml/min
Injektor: Temperatur: 200 °C
Total Flow: 84,6 ml/min
Split Flow: 81,1 ml/min (1:94,8)
Detektor: Temperatur: 250 °C
H₂ Flow: 40ml/min
Air Flow: 450 ml/min
Make-up Flow (N₂): 45 ml/min
Ofen : Temperaturprogramm: 90°C/3min mit 5°C/min auf 150°C
mit 10 °C/min auf 300 °C/ 29min
Probenvorbreitung: 1000 mg Probe + 70 mg C-12 als ISTD in 10 ml Toluol
Injektionsvolumen: 1,0 µL
Auswertung : ISTD %

**Tabelle 1:**

| **Verbindungsklasse** | **Alkoholat** | **Alkoholat** | **Onium-Verbindung** | **Alkoholat** |
|---|---|---|---|---|
| Katalysator | LiOMe | KOMe | (CH₃)₄NOH * (5 H₂O) | NaOMe |
| Temperatur [°C] | 150 | 150 | 150 | 150 |
| HCN-Umsatz [%] | 98 | 92,8 | 95 | 98,5 |
| Ausbeute (IPN in %) | 45 | 40 | 40 | 45,9 |
| Selektivität (IPN in % | 95,3 | 98,9 | 93 | 99 |
| Reaktionszeit [min] | 345 | 266 | 84 | 305 |

Fazit: NaOMe zeigt als ein homogener Katalysator bei 150 °C hinsichtlich der Ausbeute und der Selektivität eine sehr gute Performance.

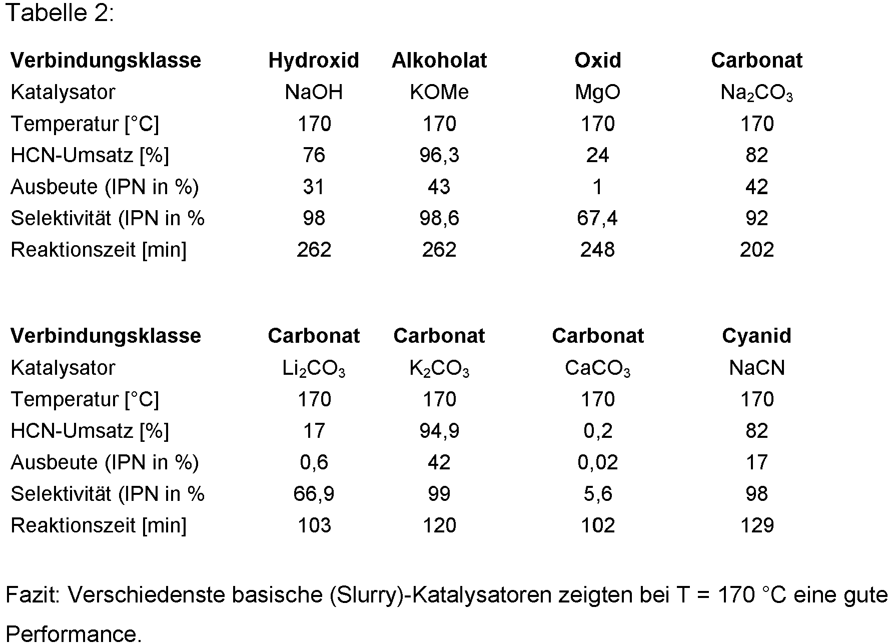

### Zu III.

In den Beispielen 1-3 wurde das Cyanid manuell zugesetzt. Dies sorgt für vergleichbare Bedingungen unter den Ergebnissen. Erfindungsgemäß bevorzugt ist allerdings die Erzeugung der Cyanidionen im Vorreaktor.

Beschreibung der kontinuierlichen Versuchsapparatur:
IPN und Ammoniak werden in einem Behälter kontinuierlich gemischt. Von dort aus gelangt die Mischung über eine Pumpe in den 2-I-Vorreaktor, der mit Ionentauscher gemäß EP 042 119 zur Katalysierung der Iminbildung aus IPN und Ammoniak gefüllt ist. Anschließend wird die Mischung in einem 6-I-Rieselbettreaktor mit drei individuell beheizbaren Temperaturzonen hydriert.
Nach der Reaktion wird das Ammoniak abgetrennt und in den Prozess zurückgeführt; zusätzlich wird verbrauchtes Ammoniak kontinuierlich ersetzt.

### Beispiel 1:

In der oben beschriebenen Versuchsapparatur wurde eine 21,5%ige ammoniakalische IPN-Lösung bei einer LHSV von 1,8 I_{Lsg} I_{Kat}⁻¹ h⁻¹ aminierend hydriert. Als Katalysator kam ein auf Kieselgur geträgerter Kobaltkontakt zum Einsatz. Der Druck in der Anlage betrug 252 bar. Das eingestellte Temperaturprofil in der Hydrierung entspricht einer adiabatischen Reaktionsführung, am Reaktoreingang betrug die Temperatur 70 °C, am Ausgang 115 °C. Das den Reaktionsteil verlassende Gemisch wurde gaschromatographisch untersucht. Die Zusammensetzung ist in Tabelle 1 wiedergegeben.

**Tabelle 1:**

| Substanz | Anteil laut GC |
|---|---|
| IPDA | 93,23 |
| IPN | 0,00 |
| IPAN | 0,71 |
| TMCA | 0,11 |
| bicycl. Amin | 4,13 |
| Amidin | 1,40 |
| Summe andere bekannte Nebenprodukte | 0,28 |
| Summe Unbekannte | 0,14 |

### Beispiel 2:

Wie Beispiel 1, jedoch wurden zusätzlich nach dem Iminierungsreaktor 40 g/h einer 10%igen wässrigen KCN-Lösung zudosiert. Dies entspricht einer Belastung von 1000 ppmw HCN bezogen auf IPN. Die Ergebnisse der gaschromatographischen Analyse des Reaktionsproduktes sind in Tabelle 2 wiedergegeben:

**Tabelle 2:**

| Substanz | Anteil laut GC |
|---|---|
| IPDA | 95,69 |
| IPN | 0,00 |
| IPAN | 0,63 |
| TMCA | 0,09 |
| bicycl. Amin | 2,03 |
| Amidin | 1,00 |
| Summe andere bekannte Nebenprodukte | 0,42 |
| Summe Unbekannte | 0,14 |

### Beispiel 3:

Wie Beispiel 1, jedoch wurden zusätzlich nach dem Iminierungsreaktor 100 g/h einer 20%igen wässrigen KCN-Lösung zudosiert. Dies entspricht einer Belastung von 5000 ppmw HCN bezogen auf IPN. Die Ergebnisse der gaschromatographischen Analyse des Reaktionsproduktes sind in Tabelle 3 wiedergegeben:

**Tabelle 3:**

| Substanz | Anteil laut GC |
|---|---|
| IPDA | 94,62 |
| IPN | 0,00 |
| IPAN | 1,15 |
| TMCA | 0,24 |
| bicycl. Amin | 2,36 |
| Amidin | 1,03 |
| Summe andere bekannte Nebenprodukte | 0,47 |
| Summe Unbekannte | 0,13 |

## Patentansprüche

1. Verfahren zur Herstellung von 3-Aminomethyl-3,5,5-trimethylcyclohexylamin, durch
I. Herstellung von Isophoron durch katalysierte Aldol-Kondensationen mit Aceton als Edukt,
Aufarbeitung des Reaktionsproduktes,
Hydrolyse des Wertstroms und Trennung in eine organische und eine wässrige Fraktion,
Gewinnung von Isophoron aus der organischen Fraktion,
destillative Aufarbeitung der wässrigen Fraktion und Weiterleitung der Brüden vom Kopf der Apparatur der destillativen Aufarbeitung in die Hydrolyse Apparatur,
wobei das Wasser aus dem Sumpf der destillativen Aufarbeitung der wässrigen Fraktion einer Entspannungsverdampfung unterworfen wird und das entstehende gereinigte Wasser in den Prozess zur Herstellung von Isophoron zurückgeführt wird;
II. Basenkatalysierte Umsetzung von Isophoron mit HCN unter Bildung von Isophoronnitril;
III. Herstellung von Isophorondiamin, mittels katalytischer Hydrierung und/oder katalytischer reduktiver Aminierung von Isophoronnitril, in Gegenwart von Ammoniak, Wasserstoff und mindestens eines Katalysators sowie gegebenenfalls eines Lösungsmittels oder Lösungsmittelgemisches, wobei die Cyanidionenkonzentration im Reaktionsgemisch, das der Hydrierung zugeführt wird von 200 ppmw bis 5000 ppmw beträgt, bezogen auf das eingesetzte Isophoronnitril, wobei die Herstellung von IPDA durch einen zweistufigen Prozess erfolgt,
und
wobei in der ersten Stufe zumindest ein Teil des eingesetzten Isophoronnitril bei An- oder Abwesenheit eines Iminierungskatalysators und/oder von Lösungsmitteln durch Reaktion mit Ammoniak in Isophoronnitrilimin umgewandelt wird, wobei der Umsatz von IPN zu IPNI nach der Iminierung größer 80 %, bevorzugt größer 90 %, besonders bevorzugt größer 95 % beträgt,
und
wobei in der zweiten Stufe das Reaktionsprodukt der ersten Stufe, so wie es anfällt oder nach einer Weiterbehandlung und/oder Zugabe weiteren Ammoniaks, unter Anwesenheit von mindestens Ammoniak und Wasserstoff und in An- oder Abwesenheit eines organischen Lösungsmittel bei einer Temperatur von 20 bis 150 °C, bevorzugt 40 bis 130 °C, und einem Druck von 0,3 bis 50 MPa, bevorzugt 5 bis 30 MPa, an Hydrierkatalysatoren aminierend hydriert wird.

2. Verfahren zur Herstellung von Isophorondiamin nach Anspruch 1, **dadurch gekennzeichnet, dass** im Schritt I. die Durchführung der Reaktion in der Flüssigphase erfolgt, wobei das Aceton durch katalytische Reaktion bei Temperaturen im Bereich von 100 bis 250 °C, bevorzugt 150 - 250 °C, besonders bevorzugt 180 - 250 °C und einem Druckbereich von 5 bis 50 bar, bevorzugt 10 - 50 bar, besonders bevorzugt von 20 - 50 bar zu Isophoron umgesetzt wird, wobei die angegebenen Werte beliebig miteinander kombiniert werden können.

3. Verfahren zur Herstellung von Isophorondiamin nach Anspruch 1, **dadurch gekennzeichnet, dass** im Schritt I. die Durchführung der Reaktion in der Gasphase erfolgt, wobei das Aceton durch katalytische Reaktion bei Temperaturen im Bereich von 100 bis 400 °C, bevorzugt 200 - 400 °C zu Isophoron umgesetzt wird.

4. Verfahren zur Herstellung von Isophorondiamin nach Anspruch 1, **dadurch gekennzeichnet, dass** im Schritt I. die Durchführung im Überkritischen Bereich erfolgt, wobei das Aceton durch katalytische Reaktion bei Temperaturen im Bereich von 250 bis 350 °C und einem Druckbereich von 50 bis 200 bar zu Isophoron umgesetzt wird.

5. Verfahren zur Herstellung von Isophorondiamin nach mindestens einem der vorherigen Ansprüche, **dadurch gekennzeichnet, dass** im Schritt I. ein homogener oder ein heterogener Katalysator, bevorzugt ein homogener Katalysator eingesetzt wird.

6. Verfahren zur Herstellung von Isophorondiamin nach mindestens einem der vorherigen Ansprüche 1 - 3, **dadurch gekennzeichnet, dass** im Schritt I. die Reaktion in der Flüssigphase mit einem homogenen Katalysator erfolgt.

7. Verfahren zur Herstellung von Isophorondiamin nach Anspruch 6, **dadurch gekennzeichnet, dass** im Schritt I. Alkalimengen von < 1 Gew.-%, bevorzugt von < 0,2 Gew.-% als Katalysator, bevorzugt NaOH in Mengen von 0,015 bis 0,05 Gew.-% eingesetzt werden.

8. Verfahren zur Herstellung von Isophorondiamin nach mindestens einem der vorherigen Ansprüche, **dadurch gekennzeichnet, dass** im Schritt I. die Abtrennung der einzelnen Fraktionen räumlich getrennt von der Isophoronsynthese durch eine Reaktivdestillation, bevorzugt in einer Reaktivdestillationskolonne, mit einer Seitenstromentnahme durchgeführt wird.

9. Verfahren zur Herstellung von Isophorondiamin nach mindestens einem der vorherigen Ansprüche, **dadurch gekennzeichnet, dass** im Schritt I. die Abtrennung in drei Fraktionen erfolgt:
a) Einer Fraktion aus nicht umgesetzten Aceton, Wasser und Leichtsiedern, die kondensiert und anschließend zur Reaktion in den Reaktor zurückgeführt wird;
b) Einer Fraktion, in der insbesondere farbgebende Substanzen angereichert werden, wobei diese Fraktion weiter aufgereinigt wird und die enthaltenden Wertstoffe im Prozess zurückgeführt werden;
c) Einer Fraktion aus Isophoron, höherkondensierten Produkten und Wasser und Katalysator, genannt Wertstoffstrom, wobei diese Fraktion anschließend einer Hydrolyse unterworfen wird.

10. Verfahren zur Herstellung von Isophorondiamin nach mindestens einem der vorherigen Ansprüche, **dadurch gekennzeichnet, dass** im Schritt I. die Fraktion a) als Brüdenstrom entnommen wird, enthaltend im Wesentlichen Aceton, Wasser und Leichtsiedern, im Wesentlichen Diacetonalkohol und Mesityloxid, kondensiert und dem Reaktor mit den Einsatzstoffen Aceton, Wasser und Katalysator wieder zugesetzt wird.

11. Verfahren zur Herstellung von Isophorondiamin nach mindestens einem der vorherigen Ansprüche, **dadurch gekennzeichnet, dass** im Schritt I. die Fraktion b) als Seitenstrom einer Destillationskolonne, bevorzugt einer Reaktivdestillationskolonne, entnommen wird.

12. Verfahren zur Herstellung von Isophorondiamin nach mindestens einem der vorherigen Ansprüche, **dadurch gekennzeichnet, dass** im Schritt I. die Aufreinigung der Fraktion b) durch eine Kombination aus Neutralisation oder Extraktion und anschließender Destillation in einer Reaktivdestillationskolonne, erfolgt,

13. Verfahren zur Herstellung von Isophorondiamin nach mindestens einem der vorherigen Ansprüche, **dadurch gekennzeichnet, dass** im Schritt I. die aufgearbeitete Phase der Fraktion b) mit den Wertprodukten aus Isophoron, Hochsiedern und gegebenenfalls Katalysator in die Hydrolyse geführt wird.

14. Verfahren zur Herstellung von Isophorondiamin nach mindestens einem der vorherigen Ansprüche, **dadurch gekennzeichnet, dass** im Schritt I. eine weitere erhaltene Phase der Fraktion b) aus Wertprodukten, im Wesentlichen enthaltend Aceton, Diacetonalkohol und Mesityloxid, in die Reaktion zurückgeführt wird.

15. Verfahren zur Herstellung von Isophorondiamin nach mindestens einem der vorherigen Ansprüche, **dadurch gekennzeichnet, dass** im Schritt I. die Fraktion c) einer Hydrolyse unterworfen wird, bei der Nebenprodukte teilweise oder vollständig in Isophoron, Aceton und andere Wertprodukte überführt.

16. Verfahren zur Herstellung von Isophorondiamin nach mindestens einem der vorherigen Ansprüche, **dadurch gekennzeichnet, dass** im Schritt I. die Hydrolyse in einer Reaktivdestillationskolonne, durchgeführt wird, in dem die entstehenden Leichtsieder, im Wesentlichen enthaltend Aceton, Diacetonalkohol und Mesityloxid, direkt aus der Hydrolysezone entfernt und in die Reaktion zurückgeführt werden.

17. Verfahren zur Herstellung von Isophorondiamin nach mindestens einem der vorherigen Ansprüche, **dadurch gekennzeichnet, dass** im Schritt I. die Hydrolyse der Fraktion c) in einem Apparat, durch eine Reaktivdestillation, durchgeführt wird, bevorzugt in einer Reaktivdestillationskolonne, wobei gleichzeitig die Trennung des Reaktionsgemisches in die Fraktionen a) bis c) erfolgt, so dass die entstehenden Produkte gleichzeitig entsprechend aufgetrennt werden und die Fraktion c) hydrolysiert wird.

18. Verfahren zur Herstellung von Isophorondiamin nach mindestens einem der vorherigen Ansprüche, **dadurch gekennzeichnet, dass** im Schritt I. die Hydrolyse c) bei Wasserkonzentration in der Hydrolyse beträgt dabei 0,1 - 99,9 Gew.-%, bevorzugt 30 - 90 Gew.%, erfolgt.

19. Verfahren zur Herstellung von Isophorondiamin nach mindestens einem der vorherigen Ansprüche, **dadurch gekennzeichnet, dass** im Schritt I. die Hydrolyse c) bei Katalysatorkonzentrationen von 0,001 - 10 Gew.-%, besonders bevorzugt von 0,05 - Gew.-%, erfolgt.

20. Verfahren zur Herstellung von Isophorondiamin nach mindestens einem der vorherigen Ansprüche, **dadurch gekennzeichnet, dass** im Schritt I. die Hydrolyse c) bei einem Druck im Hydrolysereaktor von 1 - 200 bar, bevorzugt 20 - 60 bar, besonders bevorzugt mindestens bei dem Druck, der auch im Isophoronsyntheseschritt (Reaktion) herrscht, durchgeführt wird.

21. Verfahren zur Herstellung von Isophorondiamin nach mindestens einem der vorherigen Ansprüche, **dadurch gekennzeichnet, dass** im Schritt I. die Hydrolyse c) bei einer Temperatur von 100 - 300 °C, bevorzugt 210 - 260 °C durchgeführt wird.

22. Verfahren zur Herstellung von Isophorondiamin nach mindestens einem der vorherigen Ansprüche, **dadurch gekennzeichnet, dass** im Schritt I. die Hydrolyse c) in einer Reaktivdestillationskolonne durchgeführt wird, wobei sich eine Temperatur oder ein Temperaturverlauf einstellt, der den Siedetemperaturen im Sumpf und auf den einzelnen Trenn- oder Reaktionsstufen entspricht.

23. Verfahren zur Herstellung von Isophorondiamin nach mindestens einem der vorherigen Ansprüche, **dadurch gekennzeichnet, dass** im Schritt I. die aufgearbeitete Fraktion c) und einer Phasentrennung unterworfen wird, in eine im Wesentlichen organische Fraktion d) und eine im Wesentlichen wässrige Fraktion e).

24. Verfahren zur Herstellung von Isophorondiamin nach mindestens einem der vorherigen Ansprüche, **dadurch gekennzeichnet, dass** im Schritt I. die Aufreinigung der organische Fraktion d) durch Destillation, besonders bevorzugt eine Kombination aus Neutralisation oder Extraktion und folgender Destillation, erfolgt.

25. Verfahren zur Herstellung von Isophorondiamin nach mindestens einem der vorherigen Ansprüche, **dadurch gekennzeichnet, dass** im Schritt I. die wässrige Fraktion e) einer Abwasseraufreinigung zugeführt wird, wobei die Abwasseraufreinigung bevorzugt in einer oder mehreren Destillationskolonnen durchgeführt wird.

26. Verfahren zur Herstellung von Isophorondiamin nach mindestens einem der vorherigen Ansprüche, **dadurch gekennzeichnet, dass** im Schritt I. der Druck bei der Abwasseraufreinigung in der Abwasserkolonne 1 - 200 bar, bevorzugt 20 - 60 bar, beträgt.

27. Verfahren zur Herstellung von Isophorondiamin nach mindestens einem der vorherigen Ansprüche, **dadurch gekennzeichnet, dass** im Schritt I. bei dem Systemdruck in der Abwasserkolonne gearbeitet wird, der sich im Gesamtsystem Hydrolyse/Abwasserkolonne einstellt, bevorzugt wenn die Brüden der Abwasserkolonne direkt in den Hydrolyseteil der Reaktivdestillationskolonne geleitet werden.

28. Verfahren zur Herstellung von Isophorondiamin nach mindestens einem der vorherigen Ansprüche, **dadurch gekennzeichnet, dass** im Schritt I. der Druck in der Enspannungsverdampfung unterhalb des Druckes in der Abwasserkolonne liegt.

29. Verfahren zur Herstellung von Isophorondiamin nach mindestens einem der vorherigen Ansprüche, **dadurch gekennzeichnet, dass** im Schritt I. die Brüden g) der Entspannungverdampfungsstufe, die im Wesentlichen aus reinem Wasser bestehen, kondensiert und als Wasser in die Reaktion zurückgeführt werden.

30. Verfahren zur Herstellung von Isophorondiamin nach mindestens einem der vorherigen Ansprüche, **dadurch gekennzeichnet, dass** im Schritt II. basische Alkali- oder Erdalkaliverbindungen, ausgewählt sind aus der Gruppe der Oxide, Hydroxide, Cyanide und Alkoholate, vorzugsweise der Alkoholate, organische Basen als Katalysator eingesetzt werden.

31. Verfahren zur Herstellung von Isophorondiamin gemäß einem oder mehreren der vorhergehenden Ansprüche, **dadurch gekennzeichnet, dass** im Schritt II. überschüssiges Isophoron durch Destillation abgetrennt wird und das abgetrennte Isophoron mit frischem Isophoron vermischt und in den Reaktionsreaktor zurückgeführt wird.

32. Verfahren zur Herstellung von Isophorondiamin nach mindestens einem der vorherigen Ansprüche, **dadurch gekennzeichnet, dass** im Schritt III. die Cyanidionenkonzentration durch gezielte Zudosierung von HCN oder Cyanidsalzen, oder auch durch den Einsatz von IPN-Qualitäten mit einer Cyanidionenkonzentration von 200 ppmw bis 5000ppmw, eingestellt wird.

33. Verfahren zur Herstellung von Isophorondiamin nach mindestens einem der vorherigen Ansprüche, **dadurch gekennzeichnet, dass** im Schritt III. die Einstellung der Cyanidionenkonzentration dadurch erreicht wird, dass eine gezielte Rückspaltung des IPN in der Iminierungsstufe hervorgerufen wird.

34. Verfahren zur Herstellung von Isophorondiamin nach mindestens einem der vorherigen Ansprüche, **dadurch gekennzeichnet, dass** im Schritt III. die Einstellung der Cyanidionenkonzentration dadurch erreicht, dass eine gezielte Rückspaltung des IPN in der Iminierungsstufe hervorgerufen wird, durch eine Erhöhung der Temperatur in der Iminierungsstufe um 5-50 K, bevorzugt 7-30 K, besonders bevorzugt 10-20 K oberhalb der Temperatur, die, abhängig vom Einsatz eines Iminierungskatalysators, notwendig ist, um einen Umsatz von IPN zu IPNI von mindesten 80 % in der Iminierungsstufe zu erreichen.

35. Verfahren zur Herstellung von Isophorondiamin nach mindestens einem der vorherigen Ansprüche, **dadurch gekennzeichnet, dass** im Schritt III. die Iminierungsreaktion in Gegenwart mindestens eines Iminierungskatalysators erfolgt.

36. Verfahren zur Herstellung von Isophorondiamin nach mindestens einem der vorherigen Ansprüche, **dadurch gekennzeichnet, dass** im Schritt III. die Iminierung von Isophoronnitril mit flüssigem Ammoniak ohne Zugabe weiterer Lösungsmittel durchgeführt wird, wobei in der Iminierungsstufe pro Mol eingesetztem IPN zwischen 1 und 500 Mol, bevorzugt 5 und 200 Mol, besonders bevorzugt zwischen 5 und 100 Mol Ammoniak eingesetzt werden.

37. Verfahren zur Herstellung von Isophorondiamin nach mindestens einem der vorherigen Ansprüche, **dadurch gekennzeichnet, dass** im Schritt III. die Iminierung in Gegenwart eines Suspensionskatalysators oder Festbettkatalysators, bevorzugt mindestens eines Festbettkatalysators, durchgeführt wird.

38. Verfahren zur Herstellung von Isophorondiamin nach mindestens einem der vorherigen Ansprüche, **dadurch gekennzeichnet, dass** im Schritt III. der für die Hydrierung erforderliche Wasserstoff dem Reaktor entweder im Überschuss, bevorzugt mit bis zu 10000 Moläquivalenten, zugeführt wird, oder in einer solchen Menge, dass der durch Reaktion verbrauchte Wasserstoff sowie der Teil des Wasserstoffs, der eingelöst im Produktstrom den Reaktor verlässt, nachgeführt wird.

39. Verfahren zur Herstellung von Isophorondiamin nach mindestens einem der vorherigen Ansprüche, **dadurch gekennzeichnet, dass** im Schritt III. die Hydrierung in flüssigem Ammoniak als Lösungsmittel durchgeführt wird, wobei. pro Mol IPN werden zwischen 1 und 500 Mol, bevorzugt 5 und 200 Mol, besonders bevorzugt zwischen 5 und 100 Mol Ammoniak eingesetzt werden.

40. Verfahren zur Herstellung von Isophorondiamin nach mindestens einem der vorherigen Ansprüche, **dadurch gekennzeichnet, dass** im Schritt III. als Katalysatoren Nickel-, Kupfer-, Eisen-, Palladium-, Rhodium-, Ruthenium- und Cobaltkatalysatoren, ganz besonders Ruthenium- und/oder Cobaltkatalysatoren, eingesetzt werden.

41. Verfahren zur Herstellung von Isophorondiamin nach mindestens einem der vorherigen Ansprüche, **dadurch gekennzeichnet, dass** im Schritt III. die Katalysatoren zusätzlich Dotiermetalle enthalten, bevorzugt Mo, Fe, Ag, Cr, Ni, V, Ga, In, Bi, Ti, Zr und Mn sowie die seltenen Erden.

42. Verfahren zur Herstellung von Isophorondiamin nach mindestens einem der vorherigen Ansprüche, **dadurch gekennzeichnet, dass** im Schritt III. die Katalysatoren Modifizierungsmittel enthalten, bevorzugt Alkali- und Erdalkalimetalle bzw. deren Verbindungen, bevorzugt Mg- und Ca-Verbindungen, sowie Phosphorsäure oder Schwefelsäure sowie deren Verbindungen.

43. Verfahren zur Herstellung von Isophorondiamin nach mindestens einem der vorherigen Ansprüche, **dadurch gekennzeichnet, dass** im Schritt III. Katalysatoren in Form von Pulvern oder Formkörpern, wie z. B. Extrudaten oder gepressten Pulvern, eingesetzt werden.

44. Verfahren zur Herstellung von Isophorondiamin nach mindestens einem der vorherigen Ansprüche, **dadurch gekennzeichnet, dass** im Schritt III. als Katalysatoren Vollkontakte, Raney-Typ-Katalysatoren oder Trägerkatalysatoren zur Anwendung kommen, bevorzugt Raney-Typ- und Trägerkatalysatoren.

45. Verfahren zur Herstellung von Isophorondiamin nach mindestens einem der vorherigen Ansprüche, **dadurch gekennzeichnet, dass** im Schritt III. Trägermaterialien ausgewählt aus Siliciumdioxid, Aluminiumoxid, Alumosilikate, Titandioxid, Zirkoniumdioxid, Kieselgur, Aluminium-Silicium-Mischoxide, Magnesiumoxid und Aktivkohle, eingesetzt werden.

46. Verfahren zur Herstellung von Isophorondiamin nach mindestens einem der vorherigen Ansprüche, **dadurch gekennzeichnet, dass** im Schritt III. die Hydrierkatalysatoren vor ihrem Einsatz in der Hydrierung zunächst mit Ammoniak konditioniert werden.

47. Verfahren zur Herstellung von Isophorondiamin nach mindestens einem der vorherigen Ansprüche, **dadurch gekennzeichnet, dass** im Schritt III. durch einen zweistufigen Prozess das Isophoronnitrilimin enthaltendes Gemisch mit Hilfe eines geformten Hydrierkatalysators hydriert wird.

48. Verfahren zur Herstellung von Isophorondiamin nach mindestens einem der vorherigen Ansprüche, **dadurch gekennzeichnet, dass** im Schritt III. die Hydrierung kontinuierlich in Festbettreaktoren, die in Riesel- oder Sumpffahrweise betrieben werden, durchgeführt wird.

49. Verfahren zur Herstellung von Isophorondiamin nach mindestens einem der vorherigen Ansprüche, **dadurch gekennzeichnet, dass** im Schritt III. das die Hydrierung verlassende Reaktionsgemisch einstufig oder mehrstufig aufgereinigt wird, und das Isophorondiamin erhalten wird.

50. Verfahren zur Herstellung von Isophorondiamin nach mindestens einem der vorherigen Ansprüche, **dadurch gekennzeichnet, dass** im Schritt III. das die Hydrierung verlassende Reaktionsgemisch in zwei Schritten aufgereinigt wird, wobei in einem ersten Schritt insbesondere Wasserstoff, Inertgase, Ammoniak, leicht siedende Verunreinigungen und gegebenenfalls Wasser in einer oder mehreren Destillationskolonnen ganz oder teilweise abgetrennt wird und in einem zweiten Schritt weitere leicht siedende Verunreinigungen, Wasser und schwer siedende Verunreinigungen ganz oder teilweise in Destillationskolonnen abgetrennt werden, und löp das Isophorondiamin erhalten wird.

## Claims

1. Process for preparing 3-aminomethyl-3,5,5-trimethylcyclohexylamine by
I. preparation of isophorone by catalyzed aldol condensations of acetone as a reactant,
workup of the reaction product,
hydrolysis of the stream of value and separation into an organic fraction and an aqueous fraction, recovery of isophorone from the organic fraction, distillative workup of the aqueous fraction and passage of the vapors from the top of the distillative workup apparatus onward into the hydrolysis apparatus,
wherein the water from the bottoms of the distillative workup of the aqueous fraction is subjected to a flash evaporation and the purified water which forms is recycled into the process for preparing isophorone;
II. base-catalyzed reaction of isophorone with HCN to form isophoronenitrile;
III. preparation of isophoronediamine by means of catalytic hydrogenation and/or catalytic reductive amination of isophoronenitrile, in the presence of ammonia, hydrogen and at least one catalyst and optionally a solvent or solvent mixture, where the cyanide ion concentration in the reaction mixture which is sent to the hydrogenation is 200 ppmw to 5000 ppmw, based on the isophoronenitrile used,
wherein IPDA is prepared by a two-stage process, and
wherein at least some of the isophoronenitrile used is converted in the first stage by reaction with ammonia in the presence or absence of an imination catalyst and/or of solvents to isophoronenitrileimine, the conversion of IPN to IPNI after the imination being greater than 80%, preferably greater than 90%, more preferably greater than 95%,
and
wherein the first stage reaction product, as obtained or after a further treatment and/or addition of further ammonia, is subjected in the second stage to aminating hydrogenation over hydrogenation catalysts in the presence of at least ammonia and hydrogen and in the presence or absence of an organic solvent at a temperature of 20 to 150°C, preferably 40 to 130°C, and a pressure of 0.3 to 50 MPa, preferably 5 to 30 MPa.

2. Process for preparing isophoronediamine according to Claim 1, **characterized in that** the reaction in step I. is performed in the liquid phase, the acetone being converted to isophorone by catalytic reaction at temperatures in the range from 100 to 250°C, preferably 150 - 250°C and more preferably 180 - 250°C, and a pressure range of 5 to 50 bar, preferably 10 - 50 bar and more preferably of 20 - 50 bar, it being possible to combine the values specified as desired.

3. Process for preparing isophoronediamine according to Claim 1, **characterized in that** the reaction in step I. is performed in the gas phase, the acetone being converted to isophorone by catalytic reaction at temperatures in the range from 100 to 400 °C and preferably 200-400°C.

4. Process for preparing isophoronediamine according to Claim 1, **characterized in that** step I. is performed in the supercritical range, the acetone being converted to isophorone by catalytic reaction at temperatures in the range from 250 to 350 °C and a pressure range of 50 to 200 bar.

5. Process for preparing isophoronediamine according to at least one of the preceding claims, **characterized in that** a homogeneous or heterogeneous catalyst, preferably a homogeneous catalyst, is used in step I.

6. Process for preparing isophoronediamine according to at least one of the preceding Claims 1 - 3, **characterized in that** the reaction in step I. is effected in the liquid phase with a homogeneous catalyst.

7. Process for preparing isophoronediamine according to Claim 6, **characterized in that** amounts of alkali of < 1% by weight, preferably of < 0.2% by weight, are used as catalyst in step I., preferably NaOH in amounts of 0.015 to 0.05% by weight.

8. Process for preparing isophoronediamine according to at least one of the preceding claims, **characterized in that** the removal of the individual fractions in step I. is performed spatially separately from the isophorone synthesis by a reactive distillation, preferably in a reactive distillation column, with a sidestream withdrawal.

9. Process for preparing isophoronediamine according to at least one of the preceding claims, **characterized in that** the removal in step I. is effected in three fractions:
a) a fraction composed of unconverted acetone, water and low boilers, which is condensed and then recycled into the reactor for reaction;
b) a fraction in which colored substances in particular are enriched, this fraction being purified further and the materials of value present being recycled into the process;
c) a fraction composed of isophorone, more highly condensed products and water and catalyst, called material of value stream, this fraction subsequently being subjected to a hydrolysis.

10. Process for preparing isophoronediamine according to at least one of the preceding claims, **characterized in that** fraction a) in step I. is withdrawn as a vapor stream comprising essentially acetone, water and low boilers, essentially diacetone alcohol and mesityl oxide, condensed and added again to the reactor with the acetone, water and catalyst feedstocks.

11. Process for preparing isophoronediamine according to at least one of the preceding claims, **characterized in that** fraction b) in step I. is withdrawn as a sidestream of a distillation column, preferably of a reactive distillation column.

12. Process for preparing isophoronediamine according to at least one of the preceding claims, **characterized in that** fraction b) in step I. is purified by a combination of neutralization or extraction and subsequent distillation in a reactive distillation column.

13. Process for preparing isophoronediamine according to at least one of the preceding claims, **characterized in that** the worked-up phase of fraction b) in step I. is conducted into the hydrolysis with the products of value composed of isophorone and high boilers, with or without catalyst.

14. Process for preparing isophoronediamine according to at least one of the preceding claims, **characterized in that** any further phase of fraction b) obtained, composed of products of value essentially comprising acetone, diacetone alcohol and mesityl oxide, is recycled into the reaction in step I.

15. Process for preparing isophoronediamine according to at least one of the preceding claims, **characterized in that** fraction c) in step I. is subjected to a hydrolysis in which by-products are converted partly or fully to isophorone, acetone and other products of value.

16. Process for preparing isophoronediamine according to at least one of the preceding claims, **characterized in that** the hydrolysis in step I. is performed in a reactive distillation column in which the low boilers formed, essentially comprising acetone, diacetone alcohol and mesityl oxide, are removed directly from the hydrolysis zone and recycled into the reaction.

17. Process for preparing isophoronediamine according to at least one of the preceding claims, **characterized in that** the hydrolysis of fraction c) in step I. is performed in an apparatus, by a reactive distillation, preferably in a reactive distillation column, with simultaneous separation of the reaction mixture into fractions a) to c), such that the products formed are correspondingly separated at the same time as fraction c) is hydrolyzed.

18. Process for preparing isophoronediamine according to at least one of the preceding claims, **characterized in that** the hydrolysis c) in step I. is effected at a water concentration of 0.1 - 99.9% by weight, preferably 30 - 90% by weight.

19. Process for preparing isophoronediamine according to at least one of the preceding claims, **characterized in that** the hydrolysis c) in step I. is effected at catalyst concentrations of 0.001 - 10% by weight, more preferably of 0.05 - 1% by weight.

20. Process for preparing isophoronediamine according to at least one of the preceding claims, **characterized in that** the hydrolysis c) in step I. is performed at a pressure in the hydrolysis reactor of 1 - 200 bar, preferably 20 - 60 bar, more preferably at least at the pressure which also exists in the isophorone synthesis step (reaction).

21. Process for preparing isophoronediamine according to at least one of the preceding claims, **characterized in that** the hydrolysis c) in step I. is effected at a temperature of 100 - 300°C, preferably 210 - 260°C.

22. Process for preparing isophoronediamine according to at least one of the preceding claims, **characterized in that** the hydrolysis c) in step I. is performed in a reactive distillation column, with establishment of a temperature or temperature profile according to the boiling temperatures in the bottoms and at the individual separation or reaction stages.

23. Process for preparing isophoronediamine according to at least one of the preceding claims, **characterized in that** the worked-up fraction c) in step I. and is subjected to a phase separation into an essentially organic fraction d) and an essentially aqueous fraction e).

24. Process for preparing isophoronediamine according to at least one of the preceding claims, **characterized in that** the organic fraction d) in step I. is purified by distillation, more preferably a combination of neutralization or extraction and subsequent distillation.

25. Process for preparing isophoronediamine according to at least one of the preceding claims, **characterized in that** the aqueous fraction e) in step I. is sent to a wastewater cleaning operation, the wastewater cleaning operation preferably being performed in one or more distillation columns.

26. Process for preparing isophoronediamine according to at least one of the preceding claims, **characterized in that** the pressure in the wastewater cleaning operation in the wastewater column in step I. is 1 - 200 bar, preferably 20 - 60 bar.

27. Process for preparing isophoronediamine according to at least one of the preceding claims, **characterized in that** the system pressure employed in step I. in the wastewater column is that which is established in the overall hydrolysis/wastewater column system, preferably when the vapors of the wastewater column are passed directly into the hydrolysis section of the reactive distillation column.

28. Process for preparing isophoronediamine according to at least one of the preceding claims, **characterized in that** the pressure in the flash evaporation in step I. is below the pressure in the wastewater column.

29. Process for preparing isophoronediamine according to at least one of the preceding claims, **characterized in that** the vapors g) of the flash evaporation stage consisting essentially of pure water in step I. are condensed and recycled as water into the reaction.

30. Process for preparing isophoronediamine according to at least one of the preceding claims, **characterized in that** basic alkali metal or alkaline earth metal compounds, selected from the group of the oxides, hydroxides, cyanides and alkoxides, preferably the alkoxides, or organic bases are used as catalyst in step II.

31. Process for preparing isophoronediamine according to one or more of the preceding claims, **characterized in that** excess isophorone is removed in step II. by distillation and the isophorone removed is mixed with fresh isophorone and recycled into the reaction reactor.

32. Process for preparing isophoronediamine according to at least one of the preceding claims, **characterized in that** the cyanide ion concentration in step III. is adjusted by controlled metered addition of HCN or cyanide salts, or else by the use of IPN qualities having a cyanide ion concentration of 200 ppmw to 5000 ppmw.

33. Process for preparing isophoronediamine according to at least one of the preceding claims, **characterized in that** the adjustment of the cyanide ion concentration in step III. is achieved by causing controlled redissociation of IPN in the imination stage.

34. Process for preparing isophoronediamine according to at least one of the preceding claims, **characterized in that** the adjustment of the cyanide ion concentration in step III. is achieved by causing controlled redissociation of IPN in the imination stage, by an increase in the temperature in the imination stage by 5-50 K, preferably 7-30 K, more preferably 10-20 K, above the temperature which, depending on the use of an imination catalyst, is needed to achieve a conversion of IPN to IPNI of at least 80% in the imination stage.

35. Process for preparing isophoronediamine according to at least one of the preceding claims, **characterized in that** the imination reaction in step III. is effected in the presence of at least one imination catalyst.

36. Process for preparing isophoronediamine according to at least one of the preceding claims, **characterized in that** the imination of isophoronenitrile with liquid ammonia without addition of further solvent is performed in step III, wherein between 1 and 500 mol, preferably 5 and 200 mol, more preferably between 5 and 100 mol, of ammonia is used per mole of IPN used in the imination stage.

37. Process for preparing isophoronediamine according to at least one of the preceding claims, **characterized in that** the imination in step III. is performed in the presence of a suspension catalyst or fixed bed catalyst, preferably at least one fixed bed catalyst.

38. Process for preparing isophoronediamine according to at least one of the preceding claims, **characterized in that** the hydrogen required for the hydrogenation in step III. is supplied to the reactor either in excess, preferably at up to 10 000 molar equivalents, or in such an amount that the hydrogen consumed by reaction and the portion of the hydrogen which leaves the reactor dissolved in the product stream is replenished.

39. Process for preparing isophoronediamine according to at least one of the preceding claims, **characterized in that** the hydrogenation in step III. is performed in liquid ammonia as solvent, using between 1 and 500 mol, preferably 5 and 200 mol, more preferably between 5 and 100 mol, of ammonia per mole of IPN.

40. Process for preparing isophoronediamine according to at least one of the preceding claims, **characterized in that** the catalysts used in step III. are nickel, copper, iron, palladium, rhodium, ruthenium and cobalt catalysts, very particularly ruthenium and/or cobalt catalysts.

41. Process for preparing isophoronediamine according to at least one of the preceding claims, **characterized in that** the catalysts in step III. additionally comprise doping metals, preferably Mo, Fe, Ag, Cr, Ni, V, Ga, In, Bi, Ti, Zr and Mn and the rare earths.

42. Process for preparing isophoronediamine according to at least one of the preceding claims, **characterized in that** the catalysts in step III. comprise modifiers, preferably alkali metals and alkaline earth metals or compounds thereof, preferably magnesium and calcium compounds, and phosphoric acid or sulfuric acid and compounds thereof.

43. Process for preparing isophoronediamine according to at least one of the preceding claims, **characterized in that** catalysts are used in step III. in the form of powders or shaped bodies, for example extrudates or compressed powders.

44. Process for preparing isophoronediamine according to at least one of the preceding claims, **characterized in that** the catalysts employed in step III. are unsupported catalysts, Raney-type catalysts or supported catalysts, preferably Raney-type and supported catalysts.

45. Process for preparing isophoronediamine according to at least one of the preceding claims, **characterized in that** support materials selected from silicon dioxide, aluminum oxide, aluminosilicates, titanium dioxide, zirconium dioxide, kieselguhr, aluminum-silicon mixed oxides, magnesium oxide and activated carbon are used in step III.

46. Process for preparing isophoronediamine according to at least one of the preceding claims, **characterized in that** the hydrogenation catalysts in step III. are first conditioned with ammonia before they are used in the hydrogenation.

47. Process for preparing isophoronediamine according to at least one of the preceding claims, **characterized in that** the mixture comprising isophoronenitrileimine is hydrogenated in step III. by a two-stage process with the aid of a shaped hydrogenation catalyst.

48. Process for preparing isophoronediamine according to at least one of the preceding claims, **characterized in that** the hydrogenation in step III. is performed continuously in fixed bed reactors which are operated in trickle mode or liquid phase mode.

49. Process for preparing isophoronediamine according to at least one of the preceding claims, **characterized in that** the reaction mixture leaving the hydrogenation in step III. is purified in one or more stages, and the isophoronediamine is obtained.

50. Process for preparing isophoronediamine according to at least one of the preceding claims, **characterized in that** the reaction mixture leaving the hydrogenation in step III. is purified in two steps, with complete or partial removal particularly of hydrogen, inert gases, ammonia, low-boiling impurities and optionally water in one or more distillation columns in a first step, and complete or partial removal of further low-boiling impurities, water and high-boiling impurities in distillation columns in a second step, and the isophoronediamine is obtained.

## Revendications

1. Procédé de préparation de 3-aminométhyl-3,5,5-triméthylcyclohexylamine, par
I. la préparation d'isophorone par condensation d'aldol catalysée avec de l'acétone en tant que réactif,
traitement du produit de réaction,
hydrolyse du courant de valeur et séparation en une fraction organique et une fraction aqueuse,
obtention de l'isophorone à partir de la fraction organique,
traitement distillatif de la fraction aqueuse et transfert des vapeurs depuis la tête de l'appareil du traitement distillatif dans l'appareil d'hydrolyse,
l'eau issue du fond du traitement distillatif de la fraction aqueuse étant soumise à une évaporation avec détente et l'eau purifiée formée étant recyclée dans le procédé de préparation d'isophorone ;
II. la réaction catalysée par une base de l'isophorone avec HCN avec formation d'isophorone-nitrile ;
III. la préparation d'isophorone-diamine, par hydrogénation catalytique et/ou amination réductrice catalytique d'isophorone-nitrile en présence d'ammoniac, d'hydrogène et d'au moins un catalyseur, ainsi qu'éventuellement un solvant ou un mélange de solvants, la concentration en ions cyanure dans le mélange réactionnel qui est introduit dans l'hydrogénation étant de 200 ppm en poids à 5 000 ppm en poids, par rapport à l'isophorone-nitrile utilisé, la préparation d'IPDA ayant lieu par un procédé à deux étapes, et
lors de la première étape, au moins une partie de l'isophorone-nitrile utilisé étant transformée en isophorone-nitrile-imine par réaction avec de l'ammoniac en présence ou en l'absence d'un catalyseur d'imination et/ou de solvants, la conversion de l'IPN en IPNI après l'imination étant supérieure à 80 %, de préférence supérieure à 90 %, de manière particulièrement préférée supérieure à 95 %,
et
lors de la seconde étape, le produit de réaction de la première étape, tel que formé ou après un traitement ultérieur et/ou un ajout d'ammoniac supplémentaire, étant soumis à une hydrogénation aminante sur des catalyseurs d'hydrogénation, en présence au moins d'ammoniac et d'hydrogène et en présence ou en l'absence d'un solvant organique, à une température de 20 à 150 °C, de préférence de 40 à 130 °C, et à une pression de 0,3 à 50 MPa, de préférence de 5 à 30 MPa.

2. Procédé de préparation d'isophorone-diamine selon la revendication 1, **caractérisé en ce qu'**à l'étape I, la réalisation de la réaction a lieu dans la phase liquide, l'acétone étant transformée en isophorone par réaction catalytique à des températures dans la plage allant de 100 à 250 °C, de préférence de 150 à 250 °C, de manière particulièrement préférée de 180 à 250 °C, et dans une plage de pression allant de 5 à 50 bar, de préférence de 10 à 50 bar, de manière particulièrement préférée de 20 à 50 bar, les valeurs indiquées pouvant être combinées de manière quelconque les unes avec les autres.

3. Procédé de préparation d'isophorone-diamine selon la revendication 1, **caractérisé en ce qu'**à l'étape I, la réalisation de la réaction a lieu dans la phase gazeuse, l'acétone étant transformée en isophorone par réaction catalytique à des températures dans la plage allant de 100 à 400 °C, de préférence de 200 à 400 °C.

4. Procédé de préparation d'isophorone-diamine selon la revendication 1, **caractérisé en ce qu'**à l'étape I, la réalisation a lieu dans la plage supercritique, l'acétone étant transformée en isophorone par réaction catalytique à des températures dans la plage allant de 250 à 350 °C et dans une plage de pression allant de 50 à 200 bar.

5. Procédé de préparation d'isophorone-diamine selon au moins l'une quelconque des revendications précédentes, **caractérisé en ce qu'**à l'étape I, un catalyseur homogène ou hétérogène, de préférence un catalyseur homogène, est utilisé.

6. Procédé de préparation d'isophorone-diamine selon au moins l'une quelconque des revendications 1 à 3 précédentes, **caractérisé en ce qu'**à l'étape I, la réaction a lieu dans la phase liquide avec un catalyseur homogène.

7. Procédé de préparation d'isophorone-diamine selon la revendication 6, **caractérisé en ce qu'**à l'étape I, des quantités d'alcali < 1 % en poids, de préférence < 0,2 % en poids sont utilisées en tant que catalyseur, de préférence NaOH en quantités de 0,015 à 0,05 % en poids.

8. Procédé de préparation d'isophorone-diamine selon au moins l'une quelconque des revendications précédentes, **caractérisé en ce qu'**à l'étape I, la séparation des fractions individuelles est réalisée séparément dans l'espace de la synthèse d'isophorone par une distillation réactive, de préférence dans une colonne de distillation réactive, munie d'un soutirage de courant latéral.

9. Procédé de préparation d'isophorone-diamine selon au moins l'une quelconque des revendications précédentes, **caractérisé en ce qu'**à l'étape I, la séparation a lieu en trois fractions :
a) une fraction constituée par de l'acétone non réagie, de l'eau et des composants de point d'ébullition faible, qui est condensée, puis recyclée dans le réacteur pour la réaction ;
b) une fraction dans laquelle des substances colorantes s'accumulent notamment, cette fraction étant davantage purifiée et les substances de valeur contenues étant recyclées dans le procédé ;
c) une fraction constituée par de l'isophorone, des produits condensés supérieurs et de l'eau et du catalyseur, nommée courant de produits de valeur, cette fraction étant ensuite soumise à une hydrolyse.

10. Procédé de préparation d'isophorone-diamine selon au moins l'une quelconque des revendications précédentes, **caractérisé en ce qu'**à l'étape I, la fraction a) est soutirée en tant que courant de vapeur, contenant essentiellement de l'acétone, de l'eau et des composants de point d'ébullition faible, essentiellement de l'alcool diacétonique et de l'oxyde de mésityle, condensée et rajoutée dans le réacteur avec les matières premières acétone, eau et catalyseur.

11. Procédé de préparation d'isophorone-diamine selon au moins l'une quelconque des revendications précédentes, **caractérisé en ce qu'**à l'étape I, la fraction b) est soutirée en tant que courant latéral d'une colonne de distillation, de préférence d'une colonne de distillation réactive.

12. Procédé de préparation d'isophorone-diamine selon au moins l'une quelconque des revendications précédentes, **caractérisé en ce qu'**à l'étape I, la purification de la fraction b) a lieu par une combinaison d'une neutralisation ou d'une extraction, puis d'une distillation dans une colonne de distillation réactive.

13. Procédé de préparation d'isophorone-diamine selon au moins l'une quelconque des revendications précédentes, **caractérisé en ce qu'**à l'étape I, la phase traitée de la fraction b) contenant les produits de valeur constitués par l'isophorone, les composants de point d'ébullition élevé et éventuellement le catalyseur est introduite dans l'hydrolyse.

14. Procédé de préparation d'isophorone-diamine selon au moins l'une quelconque des revendications précédentes, **caractérisé en ce qu'**à l'étape I, une phase obtenue supplémentaire de la fraction b) constituée de produits de valeur, contenant essentiellement de l'acétone, de l'alcool diacétonique et de l'oxyde de mésityle, est recyclée dans la réaction.

15. Procédé de préparation d'isophorone-diamine selon au moins l'une quelconque des revendications précédentes, **caractérisé en ce qu'**à l'étape I, la fraction c) est soumise à une hydrolyse, lors de laquelle les produits secondaires sont transformés en partie ou en totalité en isophorone, acétone et autres produits de valeur.

16. Procédé de préparation d'isophorone-diamine selon au moins l'une quelconque des revendications précédentes, **caractérisé en ce qu'**à l'étape I, l'hydrolyse est réalisée dans une colonne de distillation réactive, dans laquelle les composants de point d'ébullition faible formés, contenant essentiellement de l'acétone, de l'alcool diacétonique et de l'oxyde de mésityle, sont déchargés directement de la zone d'hydrolyse et recyclés dans la réaction.

17. Procédé de préparation d'isophorone-diamine selon au moins l'une quelconque des revendications précédentes, **caractérisé en ce qu'**à l'étape I, l'hydrolyse de la fraction c) est réalisée dans un appareil, par une distillation réactive, de préférence dans une colonne de distillation réactive, la séparation du mélange réactionnel en les fractions a) à c) ayant lieu simultanément, de sorte que les produits formés soient simultanément séparés de manière appropriée et que la fraction c) soit hydrolysée.

18. Procédé de préparation d'isophorone-diamine selon au moins l'une quelconque des revendications précédentes, **caractérisé en ce qu'**à l'étape I, l'hydrolyse c) a lieu à une concentration en eau dans l'hydrolyse de 0,1 à 99,9 % en poids, de préférence de 30 à 90 % en poids.

19. Procédé de préparation d'isophorone-diamine selon au moins l'une quelconque des revendications précédentes, **caractérisé en ce qu'**à l'étape I, l'hydrolyse c) a lieu à une concentration en catalyseur de 0,001 à 10 % en poids, de manière particulièrement préférée de 0,05 à 1 % en poids.

20. Procédé de préparation d'isophorone-diamine selon au moins l'une quelconque des revendications précédentes, **caractérisé en ce qu'**à l'étape I, l'hydrolyse c) est réalisée à une pression dans le réacteur d'hydrolyse de 1 à 200 bar, de préférence de 20 à 60 bar, de manière particulièrement préférée au moins à la pression qui règne également dans l'étape de synthèse d'isophorone (réaction).

21. Procédé de préparation d'isophorone-diamine selon au moins l'une quelconque des revendications précédentes, **caractérisé en ce qu'**à l'étape I, l'hydrolyse c) est réalisée à une température de 100 à 300 °C, de préférence de 210 à 260 °C.

22. Procédé de préparation d'isophorone-diamine selon au moins l'une quelconque des revendications précédentes, **caractérisé en ce qu'**à l'étape I, l'hydrolyse c) est réalisée dans une colonne de distillation réactive, une température ou une évolution de température qui correspond aux températures d'ébullition dans le fond et dans les étapes de séparation ou de réaction individuelles s'ajustant.

23. Procédé de préparation d'isophorone-diamine selon au moins l'une quelconque des revendications précédentes, **caractérisé en ce qu'**à l'étape I, la fraction c) traitée et est soumise à une séparation de phases, en une fraction essentiellement organique d) et une fraction essentiellement aqueuse e).

24. Procédé de préparation d'isophorone-diamine selon au moins l'une quelconque des revendications précédentes, **caractérisé en ce qu'**à l'étape I, la purification de la fraction organique d) a lieu par distillation, de manière particulièrement préférée une combinaison d'une neutralisation ou d'une extraction et d'une distillation ultérieure.

25. Procédé de préparation d'isophorone-diamine selon au moins l'une quelconque des revendications précédentes, **caractérisé en ce qu'**à l'étape I, la fraction aqueuse e) est introduite dans une purification de l'eau résiduaire, la purification de l'eau résiduaire étant de préférence réalisée dans une ou plusieurs colonnes de distillation.

26. Procédé de préparation d'isophorone-diamine selon au moins l'une quelconque des revendications précédentes, **caractérisé en ce qu'**à l'étape I, la pression lors de la purification de l'eau résiduaire dans la colonne d'eau résiduaire est de 1 à 200 bar, de préférence de 20 à 60 bar.

27. Procédé de préparation d'isophorone-diamine selon au moins l'une quelconque des revendications précédentes, **caractérisé en ce qu'**à l'étape I, on travaille à la pression du système dans la colonne d'eau résiduaire qui s'ajuste dans l'ensemble du système hydrolyse/colonne d'eau résiduaire, de préférence lorsque les vapeurs de la colonne d'eau résiduaire sont conduites directement dans la partie hydrolyse de la colonne de distillation réactive.

28. Procédé de préparation d'isophorone-diamine selon au moins l'une quelconque des revendications précédentes, **caractérisé en ce qu'**à l'étape I, la pression dans l'évaporation avec détente se situe en dessous de la pression dans la colonne d'eau résiduaire.

29. Procédé de préparation d'isophorone-diamine selon au moins l'une quelconque des revendications précédentes, **caractérisé en ce qu'**à l'étape I, les vapeurs g) de l'étape d'évaporation avec détente, qui sont essentiellement constituées d'eau pure, sont condensées et recyclées dans la réaction sous la forme d'eau.

30. Procédé de préparation d'isophorone-diamine selon au moins l'une quelconque des revendications précédentes, **caractérisé en ce qu'**à l'étape II, des composés alcalins ou alcalino-terreux basiques, choisis dans le groupe constitué par les bases organiques oxydes, hydroxydes, cyanures et alcoolates, de préférence alcoolates, sont utilisés en tant que catalyseur.

31. Procédé de préparation d'isophorone-diamine selon une ou plusieurs des revendications précédentes, **caractérisé en ce qu'**à l'étape II, l'isophorone en excès est séparée par distillation, et l'isophorone séparée est mélangée avec de l'isophorone fraîche et recyclée dans le réacteur de réaction.

32. Procédé de préparation d'isophorone-diamine selon au moins l'une quelconque des revendications précédentes, **caractérisé en ce qu'**à l'étape III, la concentration des ions cyanure est ajustée par ajout ciblé d'HCN ou de sels de cyanure, ou également par l'utilisation de qualités d'IPN ayant une concentration en ions cyanure de 200 ppm en poids à 5 000 ppm en poids.

33. Procédé de préparation d'isophorone-diamine selon au moins l'une quelconque des revendications précédentes, **caractérisé en ce qu'**à l'étape III, l'ajustement de la concentration en ions cyanure est obtenue en provoquant un rétroclivage ciblé de l'IPN dans l'étape d'imination.

34. Procédé de préparation d'isophorone-diamine selon au moins l'une quelconque des revendications précédentes, **caractérisé en ce qu'**à l'étape III, l'ajustement de la concentration en ions cyanure est obtenue en provoquant un rétroclivage cible de l'IPN dans l'étape d'imination par une élévation de la température dans l'étape d'imination de 5 à 50 K, de préférence de 7 à 30 K, de manière particulièrement préférée de 10 à 20 K au-dessus de la température qui est nécessaire, en fonction de l'utilisation d'un catalyseur d'imination, pour atteindre une conversion de l'IPN en IPNI d'au moins 80 % dans l'étape d'imination.

35. Procédé de préparation d'isophorone-diamine selon au moins l'une quelconque des revendications précédentes, **caractérisé en ce qu'**à l'étape III, la réaction d'imination a lieu en présence d'au moins un catalyseur d'imination.

36. Procédé de préparation d'isophorone-diamine selon au moins l'une quelconque des revendications précédentes, **caractérisé en ce qu'**à l'étape III, l'imination d'isophorone-nitrile avec de l'ammoniac liquide est réalisée sans ajout d'un solvant supplémentaire, entre 1 et 500 moles, de préférence entre 5 et 200 moles, de manière particulièrement préférée entre 5 et 100 moles d'ammoniac étant utilisées à l'étape d'imination par mole d'IPN utilisé.

37. Procédé de préparation d'isophorone-diamine selon au moins l'une quelconque des revendications précédentes, **caractérisé en ce qu'**à l'étape III, l'imination est réalisée en présence d'un catalyseur en suspension ou d'un catalyseur en lit fixe, de préférence d'au moins un catalyseur en lit fixe.

38. Procédé de préparation d'isophorone-diamine selon au moins l'une quelconque des revendications précédentes, **caractérisé en ce qu'**à l'étape III, l'hydrogène nécessaire pour l'hydrogénation est introduit dans le réacteur soit en excès, de préférence à hauteur de jusqu'à 10 000 équivalents molaires, soit en une quantité qui remplace l'hydrogène consommé par la réaction et la partie de l'hydrogène qui quitte le réacteur dissous dans le courant de produit.

39. Procédé de préparation d'isophorone-diamine selon au moins l'une quelconque des revendications précédentes, **caractérisé en ce qu'**à l'étape III, l'hydrogénation est réalisée dans de l'ammoniac liquide en tant que solvant, entre 1 et 500 moles, de préférence entre 5 et 200 moles, de manière particulièrement préférée entre 5 et 100 moles d'ammoniac étant utilisées par mole d'IPN.

40. Procédé de préparation d'isophorone-diamine selon au moins l'une quelconque des revendications précédentes, **caractérisé en ce qu'**à l'étape III, des catalyseurs de nickel, cuivre, fer, palladium, rhodium, ruthénium et cobalt sont utilisés en tant que catalyseurs, tout particulièrement des catalyseurs de ruthénium et/ou de cobalt.

41. Procédé de préparation d'isophorone-diamine selon au moins l'une quelconque des revendications précédentes, **caractérisé en ce qu'**à l'étape III, les catalyseurs contiennent en outre des métaux dopants, de préférence Mo, Fe, Ag, Cr, Ni, V, Ga, In, Bi, Ti, Zr et Mn, ainsi que les terres rares.

42. Procédé de préparation d'isophorone-diamine selon au moins l'une quelconque des revendications précédentes, **caractérisé en ce qu'**à l'étape III, les catalyseurs contiennent des modificateurs, de préférence des métaux alcalins et alcalino-terreux ou leurs composés, de préférence des composés de Mg et Ca, ainsi que l'acide phosphorique ou l'acide sulfurique, ainsi que leurs composés.

43. Procédé de préparation d'isophorone-diamine selon au moins l'une quelconque des revendications précédentes, **caractérisé en ce qu'**à l'étape III, des catalyseurs sont utilisés sous la forme de poudres ou de corps moulés, tels que p. ex. des extrudats ou des poudres comprimées.

44. Procédé de préparation d'isophorone-diamine selon au moins l'une quelconque des revendications précédentes, **caractérisé en ce qu'**à l'étape III, des contacts entiers, des catalyseurs de type Raney ou des catalyseurs supportés sont utilisés en tant que catalyseurs, de préférence des catalyseurs de type Raney et supportés.

45. Procédé de préparation d'isophorone-diamine selon au moins l'une quelconque des revendications précédentes, **caractérisé en ce qu'**à l'étape III, des matériaux supports choisis parmi le dioxyde de silicium, l'oxyde d'aluminium, les alumosilicates, le dioxyde de titane, le dioxyde de zirconium, la diatomite, les oxydes mixtes d'aluminium et de silicium, l'oxyde de magnésium et le charbon actif sont utilisés.

46. Procédé de préparation d'isophorone-diamine selon au moins l'une quelconque des revendications précédentes, **caractérisé en ce qu'**à l'étape III, les catalyseurs d'hydrogénation sont tout d'abord conditionnés avec de l'ammoniac avant leur utilisation dans l'hydrogénation.

47. Procédé de préparation d'isophorone-diamine selon au moins l'une quelconque des revendications précédentes, **caractérisé en ce qu'**à l'étape III, le mélange contenant l'isophorone-nitrile-imine est hydrogéné à l'aide d'un catalyseur d'hydrogénation façonné par un procédé à deux étapes.

48. Procédé de préparation d'isophorone-diamine selon au moins l'une quelconque des revendications précédentes, **caractérisé en ce qu'**à l'étape III, l'hydrogénation est réalisée en continu dans des réacteurs à lit fixe, qui sont exploités en mode de ruissellement ou de fond.

49. Procédé de préparation d'isophorone-diamine selon au moins l'une quelconque des revendications précédentes, **caractérisé en ce qu'**à l'étape III, le mélange réactionnel quittant l'hydrogénation est purifié en une étape ou en plusieurs étapes, et l'isophorone-diamine est obtenue.

50. Procédé de préparation d'isophorone-diamine selon au moins l'une quelconque des revendications précédentes, **caractérisé en ce qu'**à l'étape III, le mélange réactionnel quittant l'hydrogénation est purifié en deux étapes, lors d'une première étape, notamment de l'hydrogène, des gaz inertes, de l'ammoniac, des impuretés de point d'ébullition faible et éventuellement de l'eau étant séparés en totalité ou en partie dans une ou plusieurs colonnes de distillation, et lors d'une seconde étape, d'autres impuretés de point d'ébullition faible, de l'eau et des impuretés de point d'ébullition élevé étant séparées en totalité ou en partie dans des colonnes de distillation, et l'isophorone-diamine étant obtenue.
